# EUROPEAN PATENT APPLICATION

(11) **EP 4 372 075 A1**
(43) Date of publication of application: **22.05.2024**
(21) Application number: 22842193.9
(22) Date of filing: 15.07.2022
(51) Int. Cl.: C12M 3/00, C12N 5/071, C12N 1/00, C07K 14/78

(54) **STRUCTURE AND USE THEREOF**

(30) Priority: 15.07.2021 JP 2021117466
(71) Applicant: Cellfiber Co., Ltd., Tokyo 135-0031 (JP); Hilung Inc., Kyoto-shi, Kyoto 606-8304 (JP)
(72) Inventor: GOTOH Shimpei, Kyoto-shi, Kyoto 606-8501 (JP); IKEO Satoshi, Kyoto-shi, Kyoto 606-8501 (JP); YAMAMOTO Yuuki, Kyoto-shi, Kyoto 606-8501 (JP); IKEDA Kazuhiro, Tokyo 135-0031 (JP); YANAGISAWA Yu, Tokyo 135-0031 (JP); NAGAMOTO Tetsuji, Kyoto-shi, Kyoto 606-8304 (JP)
(74) Representative: J A Kemp LLP
(86) International application number: PCT/JP2022/027787
(87) International publication number: WO 2023/286852

(57) **Abstract**

Provided is a structure with which a lung progenitor cells can proliferate while retaining an ability to differentiate into an alveolar epithelial cell and an airway epithelial cell. A structure of the present invention includes a core portion that includes a cell population, and an outer shell portion that covers at least a part of the core portion and contains a hydrogel. The structure has a storage elastic modulus of 150 Pa or more and/or the hydrogel contains alginic acid. The cell population contains an alveolar epithelial cell, an airway epithelial cell, and/or a progenitor cell thereof.

## Description

### TECHNICAL FIELD

The present invention relates to a structure and use thereof.

### BACKGROUND ART

Lung transplant is the best treatment method for pulmonary diseases such as a chronic obstructive pulmonary disease and pulmonary fibrosis. However, there is a problem in that transplantable lungs are scarce. As another method, it is proposed that lung progenitor cells capable of differentiating into alveolar epithelial cells and airway epithelial cells are used in treatment of the pulmonary disease (Non-Patent Literature 1).

### Citation List

### Non-Patent Literature

Non-Patent Literature 1: Kalpaj R. Parekh et al., "Stem cells and lung regeneration", Cell Physiology, 2020, Volume 319, Issue 4, Pages C675-C693

### SUMMARY OF INVENTION

### Technical Problem

However, a culturing method with which the lung progenitor cells proliferate while retaining an ability to differentiate into an alveolar epithelial cell and an airway epithelial cell has not been established.

Therefore, it is an object of the present invention to provide a structure with which the lung progenitor cells can proliferate while retaining an ability to differentiate into an alveolar epithelial cell and an airway epithelial cell, a structure obtained through proliferation culture of the structure containing the lung progenitor cells, or a structure containing alveolar epithelial cells and/or airway epithelial cells obtained through differentiation induction of the structure after the proliferation culture.

### Solution to Problem

To achieve the aforementioned object, a structure of the present invention (also referred to as a "first structure" hereinafter) is a structure including:
a core portion that includes a cell population; and
an outer shell portion that covers at least a part of the core portion and contains a hydrogel,
wherein the structure has a storage elastic modulus of 150 Pa or more and/or the hydrogel contains alginic acid, and
the cell population contains an alveolar epithelial cell, an airway epithelial cell, and/or a progenitor cell thereof.

A structure of the present invention (also referred to as a "second structure" hereinafter) is a structure including:
a core portion that includes a cell population; and
an outer shell portion that covers at least a part of the core portion and contains a hydrogel,
wherein the structure has a storage elastic modulus of 150 Pa or more and/or the hydrogel contains alginic acid, and
the cell population forms a construct with functions of an alveolar epithelium and/or an airway epithelium, or is capable of forming the construct after an alveolar epithelium inducing test below or an airway epithelium inducing test below:
   alveolar epithelium inducing test: culture is performed at 37°C for 4 to 21 days in the presence of an alveolar epithelium inducing medium containing 50-nmmol/l dexamethasone, 100-µmmol/l 8-Br-cAMP, 100-µmmol/l 3-isobutyl-1-methylxanthine, 3-µmmol/l CHIR99021, 10-µmmol/l SB43154, and 10-µmmol/l Y-27632;
   airway epithelium inducing test: culture is performed at 37°C for 14 to 42 days in the presence of an airway epithelium inducing medium containing 4-µg/ml heparin, 1-µmmol/l hydroxycortisone, 10-µmmol/l N-[N-(3,5-difluorophenacetyl-L-alanyl)]-(S)-phenylglycine t-butyl ester (DAPT), and 10-µmmol/l Y-27632.

A culturing method of the present invention is a culturing method for proliferation or maintenance of a lung progenitor cell, including
a proliferation step of allowing the lung progenitor cell to proliferate by culturing a structure containing the lung progenitor cell in the presence of a proliferative factor for a lung progenitor cell,
wherein the structure includes:
   a core portion that includes a cell population; and
   an outer shell portion that covers at least a part of the core portion and contains a hydrogel,
the structure has a storage elastic modulus of 150 Pa or more and/or the hydrogel contains alginic acid, and
the cell population contains the lung progenitor cell.

A production method of the present invention (also referred to as a "method for producing an alveolar epithelium" hereinafter) is a method for producing an alveolar epithelial cell, including
an induction step of inducing an alveolar epithelial cell by culturing a structure containing a cell population in the presence of an inducing factor for an alveolar epithelial cell,
wherein the structure includes:
   a core portion that includes the cell population; and
   an outer shell portion that covers at least a part of the core portion and contains a hydrogel,
the structure has a storage elastic modulus of 150 Pa or more and/or the hydrogel contains alginic acid, and
the cell population contains a lung progenitor cell, and/or is capable of forming a construct with functions of an alveolar epithelium through culture in the induction step.

A production method of the present invention (also referred to as a "method for producing an airway epithelium" hereinafter) is a method for producing an airway epithelial cell, including
an induction step of inducing an airway epithelial cell by culturing a structure containing a cell population in the presence of an inducing factor for an airway epithelial cell,
wherein the structure includes:
   a core portion that includes the cell population; and
   an outer shell portion that covers at least a part of the core portion and contains a hydrogel,
the structure has a storage elastic modulus of 150 Pa or more and/or the hydrogel contains alginic acid, and
the cell population contains a lung progenitor cell, and/or is capable of forming a construct with functions of an airway epithelium through culture in the induction step.

### Advantageous Effects of Invention

With the present invention, the lung progenitor cells can proliferate while retaining an ability to differentiate into an alveolar epithelial cell and an airway epithelial cell.

### Brief Description of Drawings

[FIG. 1] FIG. 1 is a schematic diagram illustrating a method for inducing a lung progenitor cell in Example 1.
[FIG. 2] FIG. 2 is a schematic diagram illustrating a method for producing a cell microfiber and a method for inducing an alveolar epithelial cell or an airway epithelial cell using the cell microfiber in Example 1.
[FIG. 3] FIG. 3 shows photographs illustrating proliferation of human lung progenitor cells in the cell microfibers in Example 1.
[FIG. 4] FIG. 4 shows graphs illustrating the gene expression levels in Example 1.
[FIG. 5] FIG. 5 shows photographs illustrating immunostained images of human lung progenitor cells that proliferated in the cell microfibers in Example 1.
[FIG. 6] FIG. 6 shows the results of live imaging observation on differentiation of the human lung progenitor cells in the cell microfibers into alveolar epithelial cells in Example 1.
[FIG. 7] FIG. 7 shows graphs illustrating the results of flow cytometry in Example 1.
[FIG. 8] FIG. 8 is a graph illustrating the rates of SFTPC-GFP positive cells calculated in Example 1.
[FIG. 9] FIG. 9 shows graphs illustrating the gene expression levels in Example 1.
[FIG. 10] FIG. 10 shows photographs illustrating immunostained images of alveolar epithelial cells in the cell microfibers in Example 1.
[FIG. 11] FIG. 11 shows photographs illustrating electron microscopic images of alveolar epithelial cells in the cell microfibers in Example 1.
[FIG. 12] FIG. 12 shows graphs illustrating the gene expression levels in Example 1.
[FIG. 13] FIG. 13 shows photographs illustrating immunostained images of airway epithelial cells in the cell microfibers in Example 1.
[FIG. 14] FIG. 14 shows photographs illustrating electron microscopic images of airway epithelial cells in the cell microfibers in Example 1.
[FIG. 15] FIG. 15 is a graph illustrating the results of a principal component analysis performed on RNA-seq data in Example 1.
[FIG. 16] FIG. 16 is a heat map illustrating comparison of the expression of genes relating to peripheral lung progenitor cells and type-II alveolar epithelial cells in Example 1.
[FIG. 17] FIG. 17 is a heat map illustrating comparison of the expression of genes relating to peripheral lung progenitor cells and airway epithelial cell markers in Example 1.
[FIG. 18A] FIG. 18A is a heat map illustrating the results of gene clusters of top-2000 genes that significantly varied between samples in Example 1.
[FIG. 18B] FIG. 18B shows graphs illustrating the results of an enrichment analysis performed on the clusters in Example 1.
[FIG. 19] FIG. 19 is a graph illustrating the relative numbers of cells after the proliferation culture in Example 1.
[FIG. 20] FIG. 20 shows schematic diagrams illustrating the lengths of sections of a structure of the present invention.

### DESCRIPTION OF EMBODIMENTS

### Definitions

The term "lung progenitor cell" as used herein means a cell that is destined to have an ability to differentiate into an alveolar epithelial cell and/or an airway epithelial cell upon an embryologically appropriate stimulus. The lung progenitor cell is a cell that expresses carboxypeptidase M (CPM), NK2 homeobox 1 (NKX2.1 or NKX2-1), SRY-box 9 (SRY (sex determining region Y)-box 9, SOX9), SRY-box 2 (SRY (sex determining region Y)-box 2, SOX2), and/or forkhead box protein 2A (FOXA2). The lung progenitor cell is preferably a cell that is positive for CPM and/or NKX2.1.

The term "alveolar epithelial cell" as used herein means an epithelial cell that is present in an alveolus of the lung. The alveolar epithelial cell is, for example, a type-I alveolar epithelial cell and/or a type-II alveolar epithelial cell.

The term "type-I alveolar epithelial cell" as used herein means an epithelial cell that has a histologically flat shape and expresses PDPN (Podoplanin), AGER (Advanced Glycosylation End-Product Specific Receptor), CAV1 (Caveolin 1), HOPX (HOP Homeobox), and/or AQP5 (Aquaporin 5).

The term "type-II alveolar epithelial cell" as used herein means an epithelial cell that produces pulmonary surfactant proteins such as SFTPC (Surfactant protein C) and SFTPB (Surfactant protein B) and expresses SFTPC (Surfactant protein C), SFTPB (Surfactant protein B), ABCA3 (ATP-binding cassette sub-family A member 3), DCLAMP (Lysosome-associated membrane glycoprotein 3), and/or SLC34A2 (Sodium-dependent phosphate transport protein 2B).

The term "airway epithelial progenitor cell" as used herein means a cell that is destined to have an ability to differentiate into an airway ciliated epithelial cell, a CFTR positive airway epithelial cell, an airway mucus-producing cell, an airway basal epithelial cell, a neuroendocrine epithelial cell, and/or a club cell upon an embryologically appropriate stimulus, and expresses FOXJ1 (Forkhead box protein J1), CFTR (Cystic fibrosis transmembrane conductance regulator), P63 (transformation-related protein 63, TP63), MUC5AC (Mucin 5AC), and/or NKX2.1.

The term "airway epithelial cell" as used herein means an epithelial cell that is present in a central airway and a peripheral airway of the lung. Examples of the airway epithelial cell include an airway ciliated epithelial cell, a club cell, an airway basal epithelial cell, an airway mucus-producing cell, a CFTR positive epithelial cell, and a neuroendocrine cell.

The term "central airway epithelial progenitor cell" as used herein means an airway ciliated epithelial cell, an airway mucus-producing cell, an airway basal epithelial cell, a neuroendocrine epithelial cell, and/or a club cell, and expresses SOX2 and NKX2.1.

The term "airway ciliated epithelial cell" as used herein means an epithelial cell that has many dynamic cilia per cell in a histological sense and an epithelial cell that has cilia classified into the "9+2" structure in a morphological sense, and expresses Sentan (SNTN), Acetylated tubulin, FOXJ1, DNAH5 (dynein axonemal heavy chain 5), and/or NKX2.1.

The term "club cell" as used herein refers to an epithelial cell that produces cell-specific proteins such as SCGB1A1 (secretoglobin family 1Amember 1) and SCGB3A2 (Secretoglobin family 3A member 2) as is the case with many club cells present in a peripheral airway of the lung.

The term "airway basal epithelial cell" as used herein refers to an epithelial cell that expresses cell-specific proteins such as KRT5 (Keratin 5), NGFR (p75 neurotrophin receptor), and p63 as is the case with many basal cells present in a region ranging from a central airway to a peripheral airway.

The term "airway mucus-producing cell" as used herein refers to an epithelial cell that is large in number in a region ranging from a central airway to a peripheral airway and expresses or produces cell-specific proteins such as MUC5AC, AGR2 (Anterior gradient protein 2 homolog), and SPDEF (SAM pointed domain-containing Ets transcription factor) as is the case with a goblet cell.

The term "pluripotent cell" as used herein means a cell that has an ability to differentiate into an ectoderm cell, a mesoderm cell, and an endoderm cell. In the case where the pluripotent cell has an ability to self-replicate, the pluripotent cell can also be considered as a pluripotent stem cell.

The term "definitive endoderm (DE) cell" as used herein means a cell that is destined to have an ability to differentiate into a thymus; digestive organs such as the stomach, the intestine, and the liver; respiratory organs such as the trachea, the bronchus, and the lung; and urinary organs such as the bladder and the urethra upon an embryologically appropriate stimulus, and expresses SOX17 (SRY (sex determining region Y)-box 17) and FOXA2 (Forkhead box protein A2).

The term "anterior foregut endoderm (AFE) cell" (also referred to as an "anterior foregut cell") as used herein means a cell that is destined to have an ability to differentiate into a thymus; and respiratory organs such as the trachea, the bronchus, and the lung upon an embryologically appropriate stimulus, and expresses SOX2, SOX17, and FOXA2.

The term "ventral anterior foregut endoderm (VAFE) cell" (also referred to as a "ventral anterior foregut cell") as used herein means a cell that is destined to have an ability to differentiate into the thyroid gland and the lung upon an embryologically appropriate stimulus, and expresses NKX2.1, GATA6 (GATA-binding factor 6), and HOPX (Homeodomain-only protein).

The term "hydrogel" as used herein means a polymeric substance that contains water and has a three-dimensional structure, or a swollen form thereof.

The term "cell population" as used herein means a group of cells that includes a desired cell and is constituted by one or more cells. In the cell population, a rate (also referred to as "purity") of the desired cell in all the cells can be quantified as, for example, a rate of a cell that expresses one or more markers expressed by the desired cell. The purity is, for example, a rate in viable cells. The purity can be measured using, for example, a technique such as flow cytometry, an immunohistochemical technique, or in-situ hybridization. The purity of the desired cell in the cell population is, for example, 5% or more, 10% or more, 20% or more, 30% or more, 40% or more, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more.

The term "positive (+)" as used herein means that a higher signal is detected in an analysis method such as flow cytometry in which an antigen-antibody reaction is utilized, compared with a negative control cell that does not express the antigen, or a negative control reaction in which an antibody that does not react with the antigen is used.

The term "negative (-)" as used herein means that a lower or equal signal is detected in an analysis method such as flow cytometry in which an antigen-antibody reaction is utilized, compared with a negative control cell that does not express the antigen or a negative control reaction in which an antibody that does not react with the antigen is used.

The term "isolated" as used herein means a state in which a substance is identified and separated, and/or a state in which a substance is collected from components in a natural state. The "isolation" can be achieved by, for example, obtaining at least one purification step.

Information on the sequences of proteins described in the present specification or the sequences of nucleic acids (e.g., DNAs or RNAs) coding for the proteins is available from Protein Data Bank, UniProt, Genbank, or the like.

### Structure

A certain aspect of the present invention provides a structure with which the lung progenitor cells can proliferate while retaining an ability to differentiate into an alveolar epithelial cell and an airway epithelial cell, a structure obtained through proliferation culture of the structure containing the lung progenitor cells, or a structure containing alveolar epithelial cells and/or airway epithelial cells obtained through differentiation induction of the structure after the proliferation culture. The first structure of the present invention includes: a core portion that includes a cell population; and an outer shell portion that covers at least a part of the core portion and contains a hydrogel, wherein the structure has a storage elastic modulus of 150 Pa or more and/or the hydrogel contains alginic acid, and the cell population contains an alveolar epithelial cell, an airway epithelial cell, and/or a progenitor cell thereof. The second structure of the present invention includes: a core portion that includes a cell population; and an outer shell portion that covers at least a part of the core portion and contains a hydrogel, wherein the structure has a storage elastic modulus of 150 Pa or more and/or the hydrogel contains alginic acid, and the cell population forms a construct with functions of an alveolar epithelium and/or an airway epithelium, or is capable of forming the construct after an alveolar epithelium inducing test below or an airway epithelium inducing test below.

Alveolar epithelium inducing test: culture is performed at 37°C for 4 to 21 days in the presence of an alveolar epithelium inducing medium containing 50-nmmol/l dexamethasone, 100-µmmol/l 8-Br-cAMP, 100-µmmol/l 3-isobutyl-1-methylxanthine, 3-µmmol/l CHIR99021, 10-µmmol/l SB43154, and 10-µmmol/l Y-27632;

Airway epithelium inducing test: culture is performed at 37°C for 14 to 42 days in the presence of an airway epithelium inducing medium containing 4-µg/ml heparin, 1-µmmol/l hydroxycortisone, 10-µmmol/l N-[N-(3,5-difluorophenacetyl-L-alanyl)]-(S)-phenylglycine t-butyl ester (DAPT), and 10-µmmol/l Y-27632.

As a result of extensive research, the inventors of the present invention found that, by providing the cell population containing the lung progenitor cells as the core portion and covering a part or all of the outside of the core portion with the outer shell portion that contains a hydrogel containing alginic acid and/or a hydrogel having a predetermined storage elastic modulus, the lung progenitor cells in the core portion can proliferate while retaining an ability to differentiate into an alveolar epithelial cell and an airway epithelial cell. It is assumed that this is based on the following mechanism. However, the present invention is not limited to the following assumption. When the core portion is formed using the cell population containing the lung progenitor cells and is subjected to three-dimensional culture with the core portion being covered by the outer shell portion containing the hydrogel, the lung progenitor cells proliferate. At this time, when the hydrogel has a storage elastic modulus lower than the predetermined storage elastic modulus, the hydrogel has high extensibility. Accordingly, when the volume of the cell population increases with the proliferation of the lung progenitor cells, the outer shell portion flexibly expands. Therefore, even when the cells proliferate, the cell density of the cell population in the core portion (the density of the core portion) does not greatly change, and it is possible to secure a space in which the lung progenitor cells can form organoids containing the alveolar epithelial cells or the airway epithelial cells. Accordingly, the lung progenitor cells proliferate while differentiating into the alveolar epithelial cells or the airway epithelial cells. Meanwhile, if the hydrogel is a hydrogel such as an alginic acid gel that has a storage elastic modulus higher than or equal to the predetermined storage elastic modulus, the hydrogel has low extensibility. Accordingly, even when the volume of the cell population increases with the proliferation of the lung progenitor cells, the outer shell portion hardly expands or does not substantially expand. Therefore, the cell density of the cell population in the core portion (the density of the core portion) significantly increases with the proliferation of the cells, and it is not possible to secure a space in which the lung progenitor cells can form organoids containing the alveolar epithelial cells or the airway epithelial cells. Accordingly, it is assumed that the lung progenitor cells proliferate in the state in which the lung progenitor cells do not differentiate into the alveolar epithelial cells or the airway epithelial cells, that is to say, in the state in which the lung progenitor cells retain an ability to differentiate into the alveolar epithelial cell and the airway epithelial cell.

The type of cell contained in the cell population in the core portion can be determined depending on the application of the structure. The cell population may contain only one type of cell or two or more types of cells.

When the structure is a structure to be used in proliferation culture of the lung progenitor cells or a structure after used in the proliferation culture, the cell population contains progenitor cells of the alveolar epithelial cells and/or the airway epithelial cells, or cells capable of forming a construct with the functions of an alveolar epithelium and/or an airway epithelium after the alveolar epithelium inducing test or the airway epithelium inducing test. The above-mentioned progenitor cells and the above-mentioned cells capable of forming the construct are, for example, cells having an ability to differentiate into the alveolar epithelial cell and the airway epithelial cell, and specific examples thereof include the lung progenitor cells, the ventral anterior foregut endoderm cells, the anterior foregut endoderm cells, and/or the definitive endoderm cells. The ventral anterior foregut endoderm cells, the anterior foregut endoderm cells, and the definitive endoderm cells can also be considered as progenitor cells of the lung progenitor cells. The lung progenitor cells and the progenitor cells of the lung progenitor cells can be induced from the pluripotent cells or the pluripotent stem cells. Accordingly, the lung progenitor cells and the progenitor cells of the lung progenitor cells are preferably progenitor cells induced form the pluripotent cells or the pluripotent stem cells. Examples of the pluripotent stem cells include totipotent stem cells such as induced pluripotent stem cells (iPS cells) and embryonic stem cells (ES cells); and pluripotent stem cells such as tissue stem cells (e.g., hematopoietic stem cells, neural stem cells, and mesenchymal stem cells) and somatic stem cells.

The above-mentioned progenitor cells and the above-mentioned cells capable of forming the construct are preferably cells that are positive for CPM, NKX2.1, SOX9, SOX2, and/or FOXA2, and more preferably lung progenitor cells that are positive for CPM and/or NKX2.1, because lung progenitor cells having a high ability to differentiate into the alveolar epithelial cell and the epithelial progenitor cell can be enriched. The lung progenitor cells can be isolated at high purity using, for example, a cell surface marker CPM. Accordingly, the lung progenitor cells are preferably isolated CPM positive cells. The rate of the lung progenitor cells in the cell population is, for example, 20% or more, 30% or more, 40% or more, 50% or more, 60% or more, 70% or more, 80% or more, 85% or more, 90% or more, 95% or more, 96% or more, 97% or more, 98% or more, or 99% or more.

The construct with the functions of an alveolar epithelium means a cell-containing structure that is constituted by cells and has the functions of an alveolar epithelial tissue. Examples of the functions of an alveolar epithelium include formation of a lamellar body and/or an ability to secrete a pulmonary surfactant. Examples of the pulmonary surfactant include pulmonary surfactant protein (SFTPB) and pulmonary surfactant protein C (SFTPC). The functions of an alveolar epithelium can be evaluated based on whether or not the lamellar body is formed and/or whether or not the surfactant is secreted. Specifically, it is possible to evaluate whether or not the lamellar body is formed, by observing the structure using a transmission electron microscope or observing the structure stained using a lysosome stain (e.g., Lysotracker dye) in conformity with Example 1(12), which will be described later. Also, the presence or absence of the ability to secrete a pulmonary surfactant may be evaluated through immunohistological staining of the structure using an antibody against a target pulmonary surfactant, or through ELISA or Western blotting using the structure, or through proteome analysis or lipidome analysis of the culture supernatant of the structure to be evaluated, in conformity with Example 1(11), which will be described later.

The alveolar epithelium inducing test is a test for evaluating if cells capable of forming the construct with the functions of an alveolar epithelium are contained. As described above, in the alveolar epithelium test, culture is performed at 37°C for 4 to 21 days in the presence of an alveolar epithelium inducing medium containing 50-nmmol/l dexamethasone, 100-µmmol/l 8-Br-cAMP, 100-µmmol/l 3-isobutyl-1-methylxanthine, 3-µmmol/l CHIR99021, 10-µmmol/l SB43154, and 10-µmmol/l Y-27632. The composition of the alveolar epithelium inducing medium corresponds to that of a Ham's F12 medium containing 0.25% bovine albumin, 15-mmol/l HEPES, 0.8-mmol/l calcium chloride (CaCh), 5-µg/ml insulin, 5-µg/ml transferrin, 5-ng/ml selenious acid, and 50-U/ml penicillin-streptomycin. It is preferable that the alveolar epithelium inducing medium further contains 1% B-27 supplement (manufactured by Invitrogen). In the case where the construct with the functions of an alveolar epithelium is formed during the culture period, it is possible to evaluate the structure as containing cells capable of forming a construct having the function of an alveolar epithelium. Meanwhile, in the case where the construct with the functions of an alveolar epithelium is not formed during the culture period, it is possible to evaluate the structure as not containing cells capable of forming a construct having the function of an alveolar epithelium.

The construct with the functions of an airway epithelium means a cell-containing structure that is constituted by cells and has the functions of an airway epithelial tissue. The airway epithelial tissue contains airway ciliated epithelial cells having cilia. An example of the functions of an airway epithelium is ciliary motion. The functions of an airway epithelium can be evaluated, for example, based on the motility of the cilia (ciliary motility). Specifically, the ciliary motility can be evaluated, for example, based on the frequency of ciliary pulsation described in Literature 1 below. In the case where the frequency of ciliary pulsation of the cells in a structure to be evaluated during the culture period is similar to that of airway ciliated epithelial cells, it is possible to evaluate the structure to be evaluated as containing a construct with the functions of an airway epithelium. Meanwhile, in the case where the frequency of ciliary pulsation of the cells in a structure to be evaluated during the culture period is significantly lower than that of airway ciliated epithelial cells, it is possible to evaluate the structure as not containing cells capable of forming a construct with the functions of an airway epithelium.

Literature 1: Konishi S et al., Stem Cell Reports. 2016 Jan 12; 6(1): 18-25. doi: 10.1016/j.stemcr.2015.11.010.

The airway epithelium inducing test is a test for evaluating if cells capable of forming the construct with the functions of an airway epithelium are contained. As described above, in the airway epithelium inducing test, culture is performed at 37°C for 14 to 42 days in the presence of an airway epithelium inducing medium containing 4-µg/ml heparin, 1-µmmol/l hydroxycortisone, 10-µmmol/l N-[N-(3,5-difluorophenacetyl-L-alanyl)]-(S)-phenylglycine t-butyl ester (DAPT), and 10-µmmol/l Y-27632. The airway epithelium inducing medium is PneumaCult-ALI Basal Medium (manufactured by STEMCELL Technologies) supplemented with PneumaCult-ALI 10×Supplement and PneumaCult-ALI Maintenance Supplement. In the case where the construct with the functions of an airway epithelium is formed during the culture period, it is possible to evaluate the structure as containing cells capable of forming a construct with the functions of an airway epithelium. Meanwhile, in the case where the construct with the functions of an airway epithelium is not formed during the culture period, it is possible to evaluate the structure as not containing cells capable of forming a construct with the functions of an airway epithelium. An ALI medium (see https://www.med.unc.edu/marsicolunginstitute/wp-content/uploads/sites/547/2017/12/ALIBEGM-WEBONLY-Final.pdf) may also be used as the airway epithelium inducing medium.

When the structure is a structure containing alveolar epithelial cells and/or airway epithelial cells obtained through differentiation induction of the structure after the proliferation culture, the cell population contains the alveolar epithelial cells and/or the airway epithelial cells, or a construct with the functions of an alveolar epithelium and/or an airway epithelium. Examples of the alveolar epithelial cells include type-I alveolar epithelial cells and type-II alveolar epithelial cells. Examples of the airway epithelial cells include airway ciliated epithelial cells, CFTR positive airway epithelial cells, airway mucus-producing cells, airway basal epithelial cell, neuroendocrine epithelial cells, and club cells. The structure may contain one type of alveolar epithelial cell and/or airway epithelial cell, or two or more types of alveolar epithelial cells and/or airway epithelial cells. The structure may also contain cells having an ability to differentiate into an alveolar epithelial cell and an airway epithelial cell contained in the structure after the proliferation culture.

The density of the cell population in the core portion can be determined depending on the application of the structure. The lower limit of the density of the cell population in the core portion is, for example, 1×10³ cells/core portion-mL, 5 ×10³ cells/core portion-mL, 1×10⁴ cells/core portion-mL or more, 5 ×10⁴ cells/core portion-mL or more, 1×10⁵ cells/core portion-mL, or 5×10⁵ cells/core portion-mL, and preferably 1×10⁶ cells/core portion-mL, 5×10⁶ cells/core portion-mL, or 1×10⁷ cells/core portion-mL. Also, the upper limit of the density of the cell population in the core portion is, for example, 1×10¹⁰ cells/core portion-mL or 5×10⁹ cells/core portion-mL. The numerical range of the density of the cell population in the core portion can be set by employing any one of the upper limits listed above as an upper limit and any one of the lower limits listed above as a lower limit. In the case where the structure is used in the proliferation culture of lung progenitor cells, the proliferation of the lung progenitor cells can be promoted by setting the density of the cell population in the core portion to be relatively low.

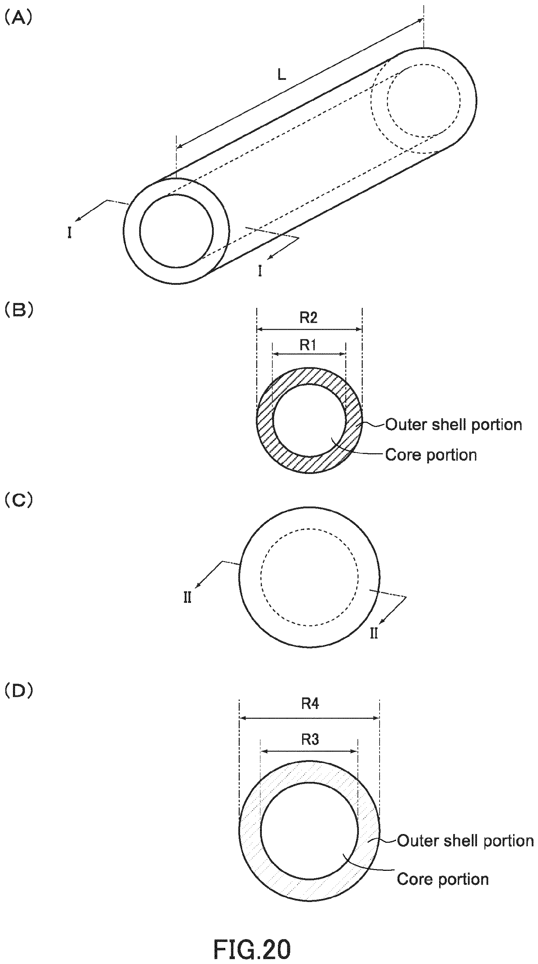

The "cells/core portion-mL" above can be calculated by dividing the number of cells in the cell population contained in the core portion of one structure by the volume of the core portion. The volume of the core portion can be calculated based on the shape of the structure. In the case where the structure is a columnar structure, the volume of the core portion can be calculated as the volume of a column whose diameter and height are the diameter of the core portion and the length in the longitudinal direction of the core portion, respectively, based on the diameter (the inner diameter of the outer shell portion; R1 in FIG. 20(B)) of the core portion and the length in the longitudinal direction of the core portion. In the case where the structure is a hollow spherical structure, the volume of the core portion can be calculated as the volume of a sphere whose diameter is the diameter of the core portion based on the diameter (the inner diameter of the outer shell portion; R3 in FIG. 20(D)) of the core portion. The diameter and the length in the longitudinal direction of the core portion each can be determined, for example, as the average value of measurement values at 10 positions placed at regular intervals. The diameter and the length in the longitudinal direction of the core portion can be measured, for example, using a phase-contrast optical microscope. The volume of the core portion may also be calculated, for example, from the production conditions of the structure. In this case, the volume of the core portion can be calculated from the discharge rate (ml/minute) and the discharge time (minute) of a cell suspension.

The cell population may be included in the core portion as isolated cells or cell masses, or cells or cell masses dispersed in a medium or the like. Preferably, the cell population is included therein as a cell suspension containing the cells or the cell masses. The cells mean cells that exist in a state in which individual cells are dispersed. The cell masses mean cell aggregates that are each formed through aggregation and/or agglutination of the cells. In the case where the cell population is dispersed in a medium or the like, the medium may be a liquid medium or a solid medium such as a gel. Specifically, it is possible to use a medium in which the cell population can be cultured, a hydrogel, which will be described later, or a mixture thereof. Unlike ordinary three-dimensional culture, the cell population can be cultured in the structure even in a state in which the cell population is not embedded in the hydrogel. Accordingly, the core portion need not contain the hydrogel.

The medium in which the cell population can be cultured can be prepared using, as a basal medium, a medium that is used to culture animal cells. Examples of the basal medium include an IMDM medium, a Medium199 medium, an Eagle's Minimum Essential Medium (EMEM) medium, an αMEM medium, a Dulbecco's modified Eagle's Medium (DMEM) medium, a Ham's F12 medium, an RPMI1640 medium, a Fischer's medium, a Neurobasal Medium (manufactured by Thermo Fisher Scientific), and mixed media obtained by mixing these media. The medium may be supplemented with serum, or may not be supplemented with serum. The medium may also contain, for example, serum alternatives such as albumin, transferrin, Knockout Serum Replacement (KSR) (a serum alternative for ES cell culture), N2 supplement (manufactured by Invitrogen), B27 supplement (manufactured by Invitrogen), a fatty acid, insulin, a collagen precursor, a trace element, 2-mercaptoethanol, and 3'-thiolglycerol. Also, the medium may contain additives such as a lipid, an amino acid, L-glutamine, Glutamax (manufactured by Invitrogen), a non-essential amino acid, a vitamin, a proliferative factor, a low-molecular compound, an antibiotic, an antioxidant, pyruvic acid, a buffer, and an inorganic salt. In the case where proliferation culture is performed using the structure, the medium is preferably an RPMI medium supplemented with B27 and an antibiotic. In the case where cells in the structure are induced into the alveolar epithelial cells or the airway epithelial cells, the medium is preferably a Ham's F12 medium supplemented with B27 and an antibiotic.

The outer shell portion contains a hydrogel. The hydrogel is, for example, alginic acid, agarose, a chitosan gel, a collagen gel, gelatin, a peptide gel, or a fibrin gel, or a mixture thereof.

The outer shell portion may contain only a hydrogel or another component in addition to a hydrogel. Examples of the other component include the medium, a culture supernatant, a growth factor, and a proliferative factor.

Regarding the storage elastic modulus of the hydrogel, from the viewpoint of enabling proliferation of the lung progenitor cells, the lower limit of the storage elastic modulus is, for example, 150 Pa or more, preferably 400 Pa or more, more preferably 800 Pa or more, and even more preferably 1 kPa or more, 5 kPa or more, or 10 kPa or more. The upper limit of the storage elastic modulus is, for example, 100 kPa or less, 50 kPa or less, or 30 kPa or less. The range of the storage elastic modulus is, for example, 150 Pa to 100 kPa, 400 Pa to 100 kPa, 800 to 100 kPa, 1 to 100 kPa, 5 to 50 kPa, or 10 to 30 kPa.

The storage elastic modulus of the hydrogel can be measured by measuring the storage elastic modulus (G') of the structure. In this specification, the storage elastic modulus (G') of the hydrogel is calculated through a measurement performed at a frequency of 1 Hz at 28°C. The storage elastic modulus (G') of the hydrogel can be measured, for example, using "Rheometer MCR302 manufactured by Anton Paar Japan K.K.". Specifically, first, the structure is rounded into a spherical shape (random coil shape) with a diameter of 8 mm, and is sandwiched between a pair of parallel disks (a jig and a stage), followed by a measurement of the dynamic elastic modulus. The storage elastic modulus (G') of the hydrogel is calculated from the obtained measurement value. Note that the gap between the jig and the stage is 2 mm.

The storage elastic modulus (G') of the hydrogel can be adjusted by changing the concentration of a hydrogel used to prepare the hydrogel, the degree of crosslinking, and/or the concentration of a material for forming a hydrogel in a solution for preparing the hydrogel. Specifically, when the concentration of a hydrogel used to prepare the hydrogel, the degree of crosslinking, and/or the concentration of a material for forming a hydrogel in a solution for preparing the hydrogel is relatively increased, the storage elastic modulus (G') of the hydrogel relatively increases. Meanwhile, when the concentration of a hydrogel used to prepare the hydrogel, the degree of crosslinking, and/or the concentration of a material for forming a hydrogel in a solution for preparing the hydrogel is relatively reduced, the storage elastic modulus (G') of the hydrogel relatively decreases.

In the structure, the outer shell portion covers a part of the core portion. That is to say, the outer shell portion covers a part or all of the outer circumferential surface of the core portion. The core portion can also be considered as a region surrounded by the outer shell portion, for example, in the state in which the core portion does not contain the cell population. The outer shell portion may be constituted by a single layer or a plurality of layers.

It is sufficient that the structure has a conformation in which the cell population can be included. For example, the structure can be designed depending on the application of the structure after the culture. As specific examples of the conformation, the structure is a columnar structure having a tubular shape or a fibrous shape; a hollow structure (e.g., bead-shaped structure) with any external shape such as a spherical shape; or the like.

In the structure, the sizes of the core portion and the outer shell portion can be determined, for example, depending on the number of cells in the cell population to be included, or the amount of the cell suspension to be included.

The length of the diameter in the cross-sectional direction orthogonal to the longitudinal direction of the core portion and the length of the inner diameter of the outer shell portion (R1 and R3 in FIG. 20) are, for example, 0.1 to 4000 µm, preferably 0.8 to 1000 µm, and more preferably 5 to 500 µm.

In the structure, it is sufficient that the thickness of the outer shell portion (a difference between R1 and R2 in FIG. 20(B), or a difference between R3 and R4 in FIG. 20(D)) is, for example, such a thickness that enables proliferation culture of the lung progenitor cells. Specifically, the thickness is 10 to 800 µm, more preferably 100 to 600 µm, and even more preferably 200 to 500 µm. When the thickness of the outer shell portion is set to 10 µm or more, the structure has a configuration in which the outer shell portion is likely to protect the cell population in the core portion. Also, when the thickness of the outer shell portion is set to 800 µm or less, the structure has, for example, a configuration in which components such as oxygen can easily permeate the outer shell portion and reach the cell population in the core portion, thus making it possible to improve the culture efficiency of the cell population. It is preferable that the thickness of the outer shell portion is substantially uniform in the structure. The term "substantially uniform" above means that differences (thicknesses) between the outer diameter and the inner diameter measured at a plurality of (e.g., ten) positions are within a range of ±10% of the average thereof. Note that the inner diameter, the outer diameter, and the thickness can be measured, for example, using a phase-contrast optical microscope.

The length (L in FIG. 20(A)) of the structure is, for example, 0.1 cm or more, preferably 5 cm or more, more preferably 10 cm or more, and even more preferably 20 cm or more. The length of the structure is, for example, 200 cm or less, preferably 150 cm or less, and more preferably 120 cm or less, because the handleability of the structure can be improved.

In an example, the structure of the present invention can be produced using the cell population-containing cell suspension, the hydrogel preparation solution, and a gelling agent.

The cell population included in the core portion may be prepared by being induced from progenitor cells such as pluripotent cells, or prepared from a biological sample collected from the lung and/or the airway. In the case where the cell population is induced from the pluripotent cells, a method for preparing the cell population can be performed in conformity with, for example, the method for inducing alveolar epithelial progenitor cells described in WO 2014/168264, the method for inducing pulmonary airway progenitor cells described in WO 2019/217429, the method for isolating lung progenitor cells described in U.S. Patent No. 10,386,368, or the method for inducing NKX2-1 lung progenitor cells described in Literature 2 below. In the case where the cell population is induced from the pluripotent cells, the obtained cell population contains the lung progenitor cells or progenitor cells of the lung progenitor cells. In the method for producing the structure of the present invention, the obtained cell population may be used as it is, or the lung progenitor cells and/or progenitor cells of the lung progenitor cells may be isolated from the obtained cell population and then used. In the case where the lung progenitor cells are isolated, the lung progenitor cells can be isolated, for example, based on the expression of CPM, NKX2.1, SOX9, SOX2, and/or FOXA2. It is preferable that the lung progenitor cells are isolated as CPM positive cells using the cell surface marker CPM.

Literature 2: Hawkins et al., J Clin Invest. 2017 Jun 1; 127(6):2277-2294. doi: 10.1172/JCI89950.

In the method for producing the structure of the present invention, the cell suspension can be prepared by suspending the cell population in a medium at a predetermined concentration. Regarding the density of the cell population in the cell suspension, the above-described examples of the density of the cell population in the core portion can be referred to. In the method for producing the structure of the present invention, the density of the cell population in the cell suspension is substantially the same as the density of the cell population in the core portion in the produced structure. Accordingly, the density of the cell population in the core portion immediately after the production can also be considered as the density of the cell population in the cell suspension.

The hydrogel preparation solution contains a material of a hydrogel that gelates due to contact with a gelling agent. For example, a case where the hydrogel is an alginic acid gel will be described as an example, but other hydrogels can also be prepared in the same manner by changing a combination of the material of a hydrogel and a gelling agent (cross-linking agent).

In the case where the hydrogel is an alginic acid gel, the hydrogel preparation solution is an alginic acid solution, and the gelling agent is a divalent metal ion. The alginic acid is partially cross-linked via the divalent metal ions to form an alginic acid gel.

The alginic acid solution is, for example, a solution prepared using an alginic acid salt such as sodium alginate, potassium alginate, or ammonium alginate, or a combination thereof. The alginic acid may be extracted natural alginic acid or a chemically modified alginic acid. An example of the chemically modified alginic acid is methacrylate-modified alginic acid or the like. The weight of an alginic acid salt with respect to the weight of a solvent in the alginic acid solution is, for example, 0.2 wt% or more, preferably 0.25 wt% or more, more preferably 0.5 wt% or more, and even more preferably 1 wt% or more. In addition to the alginic acid or a salt thereof, agar, agarose, polyethylene glycol (PEG), polylactic acid (PLA), and/or nanocellulose, etc. may also be mixed into the alginic acid solution.

The ratio (M/G ratio) between the amount of mannuronic acid (M) and the amount of guluronic acid (G) in alginic acid used to prepare the alginic acid solution is, for example, 0.4 to 1.8, and preferably 0.1 to 0.4.

The weight-average molecular weight (Mw, converted by GPC) is, for example, 700,000 to 1,000,000, and preferably 800,000 to 1,000,000.

Examples of the divalent metal ion include a calcium ion, a magnesium ion, a barium ion, a strontium ion, a zinc ion, and an iron ion, with the preferable divalent metal ion being a calcium ion or a barium ion.

It is preferable that the divalent metal ion contained in a solution is brought into contact with the alginic acid solution. In this case, the solution containing the divalent metal ion is preferably a solution containing a calcium ion, and specific examples thereof include an aqueous solution of calcium chloride, an aqueous solution of calcium carbonate, and an aqueous solution of calcium gluconate, with the preferable solution being an aqueous solution of calcium chloride.

Next, the structure is produced using the cell population-containing cell suspension, the hydrogel preparation solution, and the gelling agent. More specifically, the structure of the present invention can be produced, for example, by bringing the hydrogel preparation solution into contact with the gelling agent in a state in which the cell population-containing cell suspension is included in the hydrogel preparation solution, thereby forming a hydrogel from the hydrogel preparation solution. A method for causing the hydrogel preparation solution to include the cell suspension and a method for bringing the hydrogel preparation solution into contact with the gelling agent can be determined depending on the conformation of the structure.

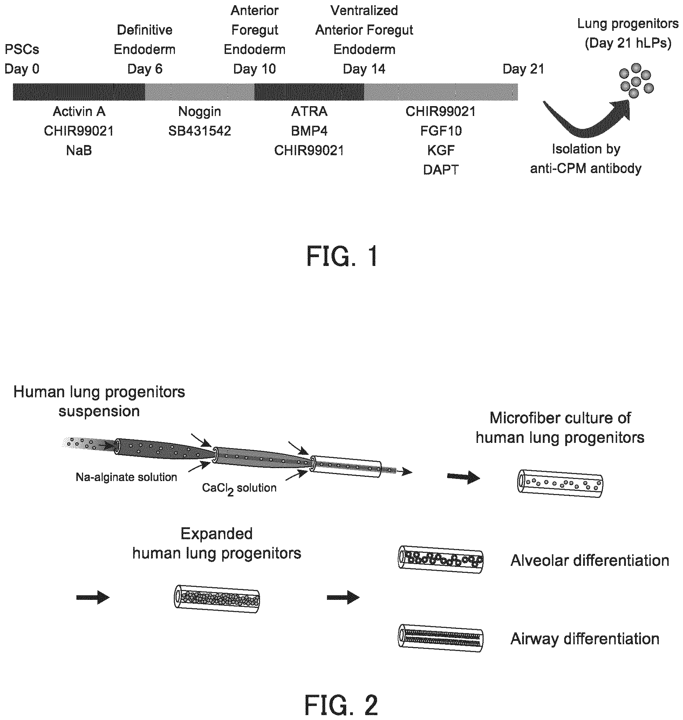

In the case where the structure is a columnar structure, the columnar structure can be produced using an apparatus and a method similar to those described in WO 2015/178427. Specifically, in a method for producing the columnar structure, a first laminar flow of the cell suspension and a second laminar flow of the hydrogel preparation solution that covers the outer circumference of the first laminar flow are formed as shown in FIG. 2. Next, in the method for producing the columnar structure, a third laminar flow of the gelling agent that covers the outer circumference of the second laminar flow is formed. Thus, in the method for producing the columnar structure, when these three laminar flows are formed, a hydrogel that covers the outer circumference of the first laminar flow is formed due to contact between the second laminar flow and the third laminar flow, and as a result, a core portion containing the cell suspension and an outer shell portion containing the hydrogel are formed.

In the case where the structure is a spherical hollow structure (e.g., hollow bead), the spherical hollow structure can be produced using an apparatus and a method similar to those described in JP 2013-71934A. Specifically, in a method for producing the spherical hollow structure, a concentric multiplex nozzle is used, and the cell suspension and the hydrogel preparation solution are simultaneously discharged from a central nozzle and an outer circumferential nozzle, respectively, to form a droplet in which the hydrogel preparation solution covers the cell suspension. In the method for producing the spherical hollow structure, a hydrogel that covers the outer circumference of the cell suspension is formed by releasing the droplet in this state into a lipid solvent containing a gelling agent, and as a result, a core portion containing the cell suspension and an outer shell portion containing the hydrogel are formed.

In the method for producing the structure of the present invention, the obtained structure can be used in proliferation culture of lung progenitor cells, a method for inducing alveolar epithelial cells, and/or a method for inducing airway epithelial cells as described later. Also, the structure of the present invention enables the proliferation culture of lung progenitor cells as described above. Accordingly, the structure can be favorably used in the proliferation culture of lung progenitor cells.

### Cell Population

Another aspect of the present invention provides a cell population isolated from a structure obtained through proliferation culture of the structure containing lung progenitor cells, or a cell population isolated from a structure containing alveolar epithelial cells and/or airway epithelial cells obtained through differentiation induction of the structure after the proliferation culture. The cell population of the present invention is a cell population isolated from the first structure and/or the second structure of the present invention.

In the case where the cell population is a cell population isolated from a structure obtained through proliferation culture of the structure containing lung progenitor cells, it is preferable that the cell population has an ability to differentiate into an alveolar epithelial cell and/or an airway epithelial cell. The ability to differentiate into the alveolar epithelial cell and/or the airway epithelial cell can be evaluated based on whether or not the alveolar epithelial cells and/or the airway epithelial cells are induced when the induction steps of a method for producing alveolar epithelial cells and a method for producing airway epithelial cells according to the present invention, which will be described later, are carried out.

Cells contained in the structure after the proliferation culture have an improved ability to differentiate into the alveolar epithelial cell, particularly the type-II alveolar epithelial cell, compared with the CPM positive lung progenitor cells. Accordingly, it is preferable that the cell population has an improved ability to differentiate into the alveolar epithelial cell, particularly the type-II alveolar epithelial cell, compared with the CPM positive lung progenitor cells.

### Construct

Another aspect of the present invention provides a construct having a conformation with the functions of an alveolar epithelium and/or an airway epithelium obtained through differentiation induction of the structure after the proliferation culture. The construct of the present invention is a construct isolated from the first structure and/or the second structure of the present invention.

In the case where the construct is a construct isolated from a structure obtained by inducing differentiation into alveolar epithelial cells in the structure after the proliferation culture, it is preferable that the construct has the lamellar body, and/or has an ability to secret a pulmonary surfactant. The surfactant is, for example, pulmonary surfactant protein B and/or pulmonary surfactant protein C.

In the case where the construct is a construct isolated from a structure obtained by inducing differentiation into airway epithelial cells in the structure after the proliferation culture, it is preferable that the construct has cilia, and the cilia have ciliary motility.

### Method for Culturing Lung Progenitor Cells

Another aspect of the present invention provides a method that enables proliferation culture or maintenance culture of the lung progenitor cells. The culturing method of the present invention is a culturing method for proliferation or maintenance of a lung progenitor cell, the method including a proliferation step of allowing the lung progenitor cell to proliferate by culturing a structure containing the lung progenitor cell in the presence of a proliferative factor for a lung progenitor cell, wherein the structure includes: a core portion that includes a cell population; and an outer shell portion that covers at least a part of the core portion and contains a hydrogel, the structure has a storage elastic modulus of 150 Pa or more and/or the hydrogel contains alginic acid, and the cell population contains the lung progenitor cell.

The proliferation step is performed, for example, using a medium containing the proliferative factor for a lung progenitor cell. Regarding the medium, the above-described examples can be referred to. The medium used in the proliferation step is preferably an RPMI medium containing B-27 Supplement.

The proliferative factor for a lung progenitor cell is, for example, a GSK3β inhibitor, an FGF10 (Fibroblast Growth Factor 10), a KGF (Keratinocyte Growth Factor), a Notch inhibitor, and/or a ROCK inhibitor. The proliferative factors may be used alone or in combination of two or more. The proliferative factors are preferably used in combination of two or more, and more preferably in combination of all of them.

The GSK3β inhibitor is a substance that inhibits the kinase activity (e.g., an ability to phosphorylate β-catenin) of GSK-3β protein. Examples of the GSK3β inhibitor include indirubin derivatives such as BIO (GSK-3β inhibitor IX; 6-bromoindirubin 3'-oxime); maleimide derivatives such as SB216763 (3-(2,4-dichlorophenyl)-4-(1-methyl-1H-indol-3-yl)-1H-pyrrole-2,5-dione); phenyl α bromomethylketone compounds l such as GSK-3β inhibitor VII (4-dibromoacetophenone); cell-membrane-permeable phosphorylated peptides such as L803-mts (GSK-3β peptide inhibitor; Myr-N-GKEAPPAPPQSpP-NH2); CHIR99021 (6-[2-[4-(2,4-dichlorophenyl)-5-(4-methyl-1H-imidazol-2-yl)pyrimidin-2-ylamino]ethylamino]pyridine-3-carbonitrile), and nucleic acid molecules (e.g., siRNA, shRNA, and antisense) for suppressing expression of GSK3β protein. The GSK3β inhibitor is preferably CHIR99021 because it has high specificity for GSK3β protein.

The concentration of the GSK3β inhibitor in the medium is, for example, 1 nmol/l to 50 µmol/l, 10 nmol/l to 40 µmol/l, 50 nmol/l to 30 µmol/l, 100 nmol/l to 25 µmol/l, 500 nmol/l to 20 µmol/l, or 750 nmol/l to 15 µmol/l.

The FGF10 is a protein encoded by a polynucleotide registered in NCBI under accession number NM_004465. The FGF10 may be in the active form resulting from cleavage by a protease.

The concentration of the FGF10 in the medium is, for example, 10 ng/ml to 1 µg/ml, 20 ng/ml to 1 µg/ml, 30 ng/ml to 900 ng/ml, 40 ng/ml to 800 ng/ml, 50 ng/ml to 700 ng/ml, 60 ng/ml to 600 ng/ml, 70 ng/ml to 500 ng/ml, 80 ng/ml to 400 ng/ml, or 90 ng/ml to 300 ng/ml.

The Notch inhibitor is a substance that inhibits a Notch signal. Examples of the Notch inhibitor include DAPT (N-[2S-(3,5-difluorophenyl)acetyl]-L-alanyl-2-phenyl-1,1-dimethylethyl ester-glycine), DBZ (N-[(1S)-2-[[(7S)-6,7-Dihydro-5-methyl-6-oxo-5H-dibenz[b,d]azepin-7-yl]amino]-1-methyl-2-oxoethyl]-3,5-difluorobenzeneacetamide), Compound E (N-[(1S)-2-[[(3 S)-2,3-Dihydro-1-methyl-2-oxo-5-phenyl-1H-1,4-benzodiazepin-3-yl]amino]-1-methyl-2-oxoethyl]-3,5-difluorobenzeneacetamide), FLI-06 (Cyclohexyl 1,4,5,6,7,8-hexahydro-2,7,7-trimethyl-4-(4-nitrophenyl)-5-oxo-3-quinolinecarboxylate), LY411575 (N2-[(2S)-2-(3,5-Difluorophenyl)-2-hydroxyethanoyl]-N1-[(7S)-5-methyl-6-oxo-6,7-dihydro-5H-dibenzo[b,d]azepin-7-yl]-L-alaninamide), and nucleic acid molecules (e.g., siRNA, shRNA, and antisense) for suppressing expression of Notch protein, with the preferable Notch inhibitor being DAPT.

The concentration of the Notch inhibitor in the medium is, for example, 1 nmol/l to 50 µmol/l, 10 nmol/l to 40 µmol/l, 50 nmol/l to 30 µmol/l, 100 nmol/l to 25 µmol/l, or 500 nmol/l to 20 µmol/l.

The KGF is a protein encoded by a polynucleotide registered in NCBI under accession number NM_002009. The KGF may be in the active form resulting from cleavage by a protease.

The concentration of the KGF in the medium is, for example, 10 ng/ml to 1 µg/ml, 20 ng/ml to 1 µg/ml, 30 ng/ml to 900 ng/ml, 40 ng/ml to 800 ng/ml, 50 ng/ml to 700 ng/ml, 60 ng/ml to 600 ng/ml, 70 ng/ml to 500 ng/ml, 80 ng/ml to 400 ng/ml, or 90 ng/ml to 300 ng/ml.

The ROCK inhibitor is a substance that can inhibit the functions of Rho kinase (ROCK). Examples of the ROCK inhibitor include Y-27632 ((+)-(R)-trans-4-(1-aminoethyl)-N-(4-pyridyl)cyclohexanecarboxamide dihydrochloride), Fasudil/HA1077 (5-(1,4-diazepane-1-sulfonyl)isoquinoline), H-1152 ((S)-(+)-2-methyl-1-[(4-methyl-5-isoquinolinyl)sulfonyl]homopiperazine), Wf-536 ((+)-(R)-4-(1-aminoethyl)-N-(4-pyridyl) benzamide), and nucleic acid molecules (e.g., siRNA, shRNA, and antisense) for suppressing expression of ROCK protein, with the preferable ROCK inhibitor being Y-27632.

The concentration of the ROCK inhibitor in the medium is, for example, 1 nmol/l to 50 µmol/l, 10 nmol/l to 40 µmol/l, 50 nmol/l to 30 µmol/l, 100 nmol/l to 25 µmol/l, 500 nmol/l to 20 µmol/l, or 750 nmol/l to 15 µmol/l.

The culturing days in the proliferation step, during which the lung progenitor cells proliferate, can be set depending on the desired proliferation degree. The lower limit of the culturing days is, for example, 3 days or more, 4 days or more, 5 days or more, 6 days or more, 7 days or more, 8 days or more, 9 days or more, 10 days or more, 11 days or more, 12 days or more, or even more days. The upper limit of the culturing days is, for example, 365 days or less, 100 days or less, 90 days or less, or 80 days or less. Using the structure makes it possible to culture the lung progenitor cells, for example, for a longer period of time. The culturing days in the culturing step may be set to be a period during which the shell portion is filled with the cell population.

The common culturing conditions for the lung progenitor cells can be employed as the culturing conditions in the proliferation step. Specifically, the culture temperature is, for example, about 30 to 40°C, and preferably about 37°C. The culture can be performed, for example, under a CO₂-containing air atmosphere, and the CO₂ concentration is, for example, about 1 to 10% or about 2 to 5%. It is preferable to perform the culture in a humid environment.

With the culturing method of the present invention, the lung progenitor cells can proliferate while retaining an ability to differentiate into an alveolar epithelial cell and an airway epithelial cell by culturing the lung progenitor cells in a state of being included in the structure. Accordingly, the culturing method of the present invention enables maintenance culture and proliferation culture of the lung progenitor cells. Therefore, the culturing method of the present invention can also be considered as a method for producing cells having an ability to differentiate into an alveolar epithelial cell and/or an airway epithelial cell. Also, with the culturing method of the present invention, it is possible to improve an ability to differentiate into an alveolar epithelial cell. Accordingly, the culturing method of the present invention can also be considered as, for example, a method for improving an ability to differentiate into an alveolar epithelial cell.

### Method for Producing Alveolar Epithelial Cells

Another aspect of the present invention provides a method for producing alveolar epithelial cells using the structure. The production method of the present invention is a method for producing an alveolar epithelial cell, the method including an induction step of inducing an alveolar epithelial cell by culturing a structure containing a cell population in the presence of an inducing factor for an alveolar epithelial cell, wherein the structure includes: a core portion that includes the cell population; and an outer shell portion that covers at least a part of the core portion and contains a hydrogel, the structure has a storage elastic modulus of 150 Pa or more and/or the hydrogel contains alginic acid, and the cell population contains a lung progenitor cell, and/or is capable of forming a construct with functions of an alveolar epithelium through culture in the induction step.

The method for producing an alveolar epithelium according to the present invention may include a proliferation step of allowing the lung progenitor cells to proliferate by culturing the structure in the presence of the proliferative factor for a lung progenitor cell, prior to the induction step. The method for producing an alveolar epithelium according to the present invention includes the proliferation step, thus making it possible to induce even more alveolar epithelial cells.

The induction step is performed, for example, using a medium containing the proliferative factor of an alveolar epithelial cell. Regarding the medium, the above-described examples can be referred to. The medium used in the induction step is preferably a Ham's F12 medium containing B-27 Supplement.

The inducing factor for an alveolar epithelial cell can be selected depending on the type of alveolar epithelial cells to be induced. In the case where the alveolar epithelial cells are type-I alveolar epithelial cells, the inducing factor for an alveolar epithelial cell is an inducing factor for a type-I alveolar epithelial cell, and a specific example thereof is the above-mentioned Wnt inhibitor. The inducing factors may be used alone or in combination of two or more. The inducing factors are preferably used in combination of two or more, and more preferably in combination of all of them.

The Wnt inhibitor is a substance that inhibits a Wnt signal. Examples of the Wnt inhibitor include IWP2 (N-(6-methyl-2-benzothiazolyl)-2-(3,4,6,7-tetrahydro-4-oxo-3-phenylthieno3,2-dpyrimidin-2-yl)thio), Dickkopf-associated protein 1 (DKK1), XAV939 (3,5,7,8-tetrahydro-2-[4-(trifluoromethyl)phenyl]-4H-thiopyrano[4,3-d]-pyrimidin-4-one); and nucleic acid molecules (e.g., siRNA, shRNA, and antisense) for suppressing expression of Wnt protein, with the preferable Wnt inhibitor being XAV939.

The concentration of the Wnt inhibitor in the medium is, for example, 1 nmol/l to 50 µmol/l, 10 nmol/l to 40 µmol/l, 50 nmol/l to 30 µmol/l, 100 nmol/l to 25 µmol/l, or 500 nmol/l to 20 µmol/l.

The culturing days in the step of inducing type-I alveolar epithelial cells can be set depending on a period during which the type-I alveolar epithelial cells are induced. The lower limit of the culturing days is, for example, 4 days or more, 5 days or more, 6 days or more, 7 days or more, 8 days or more, 9 days or more, 10 days or more, 11 days or more, 12 days or more, or even more days. The upper limit of the culturing days is, for example, 35 days or less, 30 days or less, 28 days or less, or 21 days or less.

Regarding the culturing conditions in the step of inducing type-I alveolar epithelial cells, the examples of the culturing conditions in the above-described proliferation step can be referred to.

In the case where the alveolar epithelial cells are type-II alveolar epithelial cells, the inducing factor of an alveolar epithelial cell is an inducing factor for a type-II alveolar epithelial cell, and a specific example thereof is a steroid, a cAMP derivative, a phosphodiesterase inhibitor, KGF, a GSK3β inhibitor, a TGFβ inhibitor, a ROCK inhibitor, FGF10, and/or EGF (Epidermal Growth Factor). The inducing factors may be used alone or in combination of two or more. The inducing factors are preferably used in combination of two or more, and a combination of the steroid, the cAMP derivative, the phosphodiesterase inhibitor, and the KGF is more preferable. Regarding the examples of the KGF, the GSK3β inhibitor, the ROCK inhibitor, and the FGF10 and the concentrations thereof in the medium, the descriptions above can be referred to.

The steroid is a steroidal anti-inflammatory agent. Examples of the steroid include glucocorticoids and synthetic derivatives thereof, and specific examples thereof include hydrocortisone, hydrocortisone succinate, prednisolone, methylprednisolone, methylprednisolone succinate, triamcinolone, triamcinolone acetonide, dexamethasone, and betamethasone, with the preferable steroid being dexamethasone or hydrocortisone.

The concentration of the steroid in the medium is, for example, 1 nmol/l to 50 µmol/l, 10 nmol/l to 40 µmol/l, 10 nmol/l to 30 µmol/l, 10 nmol/l to 25 µmol/l, or 10 nmol/l to 20 µmol/l.

The cAMP derivative is a compound obtained by modifying cyclic AMP with a substituent (adding a substituent to cyclic AMP). Examples of the cAMP derivative include cyclic adenosine monophosphate (cAMP), 8-bromo cyclic adenosine monophosphate (8-Br-cAMP), 8-chloro cyclic adenosine monophosphate (8-Cl-cAMP), 8-(4-Chlorophenylthio)cyclic adenosine monophosphate (8-CPT-cAMP), and dibutyryl cyclic adenosine monophosphate (DB-cAMP), with the preferable cAMP derivative being 8-Br-cAMP.

The concentration of the cAMP derivative in the medium is, for example, 1 nmol/l to 50 µmol/l, 10 nmol/l to 40 µmol/l, 50 nmol/l to 30 µmol/l, 100 nmol/l to 25 µmol/l, or 500 nmol/l to 20 µmol/l.

The phosphodiesterase inhibitor is a compound that inhibits phosphodiesterase (PDE) and thus increases the intracellular concentration of cAMP or cGMP. Examples of the phosphodiesterase inhibitor include 1,3-dimethylxanthine, 6,7-dimethoxy-1-(3,4-dimethoxybenzyl)isoquinoline, 4-{[3',4'-(methylenedioxy)benzyl]amino}-6-methoxyquinazoline, 8-methoxymethyl-3-isobutyl-1-methylxanthine, and 3-isobutyl-1-methylxanthine (IBMX), with the preferable phosphodiesterase inhibitor being 1,3-dimethylxanthine or IBMX.

The concentration of the phosphodiesterase inhibitor in the medium is, for example, 1 nmol/l to 50 µmol/l, 10 nmol/l to 40 µmol/l, 50 nmol/l to 30 µmol/l, 50 nmol/l to 25 µmol/l, or 50 nmol/l to 20 µmol/l.

The TGFβ inhibitor is a substance that inhibits signaling via SMAD produced due to binding of TGFβ to a receptor. Examples of the TGFβ inhibitor include substances that inhibit binding to a TGFβ receptor ALK family, and substances that inhibit phosphorylation of SMAD by an ALK family. Specific examples of the TGFβ inhibitor include Lefty-1 (NCBI accession number: NM_010094 (mouse), NM_020997 (human)), SB431542 (4-(4-(benzo[d][1,3]dioxol-5-yl)-5-(pyridine-2-yl)-1H-imidazol-2-yl)benzamide), SB202190 (4-(4-fluorophenyl)-2-(4-hydroxyphenyl)-5-(4-pyridyl)-1H-imidazole), SB505124 (2-(5-benzo1,3dioxol-5-yl-2-tert-butyl-3H-imidazol-4-yl)-6-methylpyridine), NPC30345, SD093, SD908, SD208 (Scios), LY2109761, LY364947, LY580276(Lilly Research Laboratories), and A-83-01 (WO2009/146408), with the preferable TGFβ inhibitor being SB431542.

The concentration of the TGFβ inhibitor in the medium is, for example, 1 nmol/l to 50 µmol/l, 10 nmol/l to 40 µmol/l, 50 nmol/l to 30 µmol/l, 100 nmol/l to 25 µmol/l, or 500 nmol/l to 20 µmol/l.

The EGF is a protein encoded by a polynucleotide registered in NCBI under accession number NM_001178130, NM_001178131, NM_001963, or NM _001357021.

The concentration of the EGF in the medium is, for example, 10 ng/ml to 1 µg/ml, 20 ng/ml to 1 µg/ml, 30 ng/ml to 900 ng/ml, 40 ng/ml to 800 ng/ml, 50 ng/ml to 700 ng/ml, 60 ng/ml to 600 ng/ml, 70 ng/ml to 500 ng/ml, 80 ng/ml to 400 ng/ml, or 90 ng/ml to 300 ng/ml.

The culturing days in the step of inducing type-II alveolar epithelial cells can be set depending on a period during which the type-II alveolar epithelial cells are induced. The lower limit of the culturing days is, for example, 4 days or more, 5 days or more, 6 days or more, 7 days or more, 8 days or more, 9 days or more, 10 days or more, 11 days or more, 12 days or more, 13 days or more, 14 days or more, 15 days or more, or even more days. The upper limit of the culturing days is, for example, 35 days or less, 30 days or less, 28 days or less, or 21 days or less.

Regarding the culturing conditions in the step of inducing type-II alveolar epithelial cells, the examples of the culturing conditions in the above-described proliferation step can be referred to.

The type-I alveolar epithelial cells can also be induced from type-II alveolar epithelial cells, which will be described later. Accordingly, the method for producing alveolar epithelial cells according to the present invention may include a step of inducing type-I alveolar epithelial cells by culturing the structure in the presence of the inducing factor for a type-I alveolar epithelial cell after induction of the type-II alveolar epithelial cells.

### Method for Producing Airway Epithelial Cells

Another aspect of the present invention provides a method for producing airway epithelial cells using the structure. The production method of the present invention is a method for producing an airway epithelial cell, the method including an induction step of inducing an airway epithelial cell by culturing a structure containing a cell population in the presence of an inducing factor for an airway epithelial cell, wherein the structure includes: a core portion that includes the cell population; and an outer shell portion that covers at least a part of the core portion and contains a hydrogel, the structure has a storage elastic modulus of 150 Pa or more and/or the hydrogel contains alginic acid, and the cell population contains a lung progenitor cell, and/or is capable of forming a construct with functions of an airway epithelium through culture in the induction step.

The method for producing an airway epithelium according to the present invention may include a proliferation step of allowing the lung progenitor cells to proliferate by culturing the structure in the presence of the proliferative factor for a lung progenitor cell, prior to the induction step. The method for producing an airway epithelium according to the present invention includes the proliferation step, thus making it possible to induce even more airway epithelial cells.

The induction step is performed, for example, using a medium containing the proliferative factor for an alveolar epithelial cell. Regarding the medium, the above-described examples can be referred to. The medium used in the induction step is preferably PneumaCult-ALI Basal Medium supplemented with PneumaCult-ALI 10×Supplement and PneumaCult-ALI Maintenance Supplement.

The inducing factor for an airway epithelial cell is, for example, a steroid, a Notch inhibitor, a ROCK inhibitor, and/or heparin. The inducing factors may be used alone or in combination of two or more. The inducing factors are preferably used in combination of two or more, and more preferably in combination of all of them. Regarding the examples of the steroid, the Notch inhibitor, and the ROCK inhibitor and the concentrations thereof in the medium, the descriptions above can be referred to.

The heparin is a glycosaminoglycan having anti-coagulant effects. It is assumed that the heparin contributes to induction of the airway epithelial cells through activation of a growth factor, a cytokine, and the like. The heparin may also be a heparin derivative, and an example thereof is a heparinoid.

The concentration of the heparin in the medium is, for example, 10 ng/ml to 100 µg/ml, 20 ng/ml to 100 µg/ml, 30 ng/ml to 90 µg/ml, 40 ng/ml to 80 µg/ml, 50 ng/ml to 70 µg/ml, 60 ng/ml to 60 µg/ml, 70 ng/ml to 50 µg/ml, 80 ng/ml to 40 µg/ml, or 90 ng/ml to 30 µg/ml.

The culturing days in the induction step can be set depending on a period during which the airway epithelial cells are induced. The lower limit of the culturing days is, for example, 14 days or more, 15 days or more, 16 days or more, 17 days or more, 18 days or more, 19 days or more, 20 days or more, 21 days or more, or even more days. The upper limit of the culturing days is, for example, 49 days or less, 45 days or less, 42 days or less, 40 days or less, 35 days or less, 30 days or less, or 28 days or less.

Regarding the culturing conditions in the step of inducing airway epithelial cells, the examples of the culturing conditions in the above-described proliferation step can be referred to.

### Examples

The following describes examples of the present invention. However, the present invention is not limited to the examples below. Commercially available reagents were used based on their protocols unless otherwise stated.

### Example 1

It was checked whether the cell microfiber of the present invention enables proliferation culture of human lung progenitor cells while allowing the human lung progenitor cells to retain the lung progenitor cell properties.

### (1) Production of Human Induced Pluripotent Stem Cells Expressing Fluorescent Protein

A cell strain expressing a fluorescent protein in response to expression of surfactant protein C (SFTPC) serving as a type-II alveolar epithelial cell marker was used as human pluripotent stem cells (hPSC) to confirm differentiation from human PSC-derived lung progenitor cells (hLPs). Specifically, as human induced pluripotent stem cells (hiPSCs), the B2-3 strain (hiPSC strain B2-3) was used. The hiPSC strain B2-3 is a hiPSC in which an expression vector containing a polynucleotide coding for a promoter for the SFTPC gene and a green fluorescent protein (GFP) is knocked-in. The hiPSC strain B2-3 was produced using the method described in Reference Literature 1 below. The hiPSC strain B2-3 is derived from the hiPSC strain 201B7 (Reference Literature 2 below).

Reference Literature 1: S. Gotoh, et al., "Generation of alveolar epithelial spheroids via isolated progenitor cells from human pluripotent stem cells", Stem Cell Reports 3(3) (2014) 394-403.

Reference Literature 2: K. Takahashi, et al., "Induction of pluripotent stem cells from adult human fibroblasts by defined factors", Cell 131(5) (2007) 861-72.

Next, a red fluorescent protein (mCherry) was expressed in the hiPSC strain B2-3. Specifically, first, a pCAGGS-mCherry vector was produced by replacing the EGFP gene sequence in the pCAGGS-EGFP vector with the mCherry gene sequence in the pmCherry-C1 vector (manufactured by Clontech). The pCAGGS-mCherry vector was introduced into 1.0×10⁶ hiPSC strain B2-3 cells using electroporation gene transfer. An NEPA21 electroporator (poring pulse: pulse voltage, 125 V; pulse width, 5 ms; pulse number, 2; NEPA GENE) was used for the gene transfer. Two to three weeks after the gene transfer, mCherry positive cells were selected using flow cytometry, and thus mCherry-expressing SFTPC-GFP reporter hiPSCs (hiPSC strain mB2-3) were produced.

### (2) Method for Maintenance Culture of Human Stem Cells

In Example 1, the hiPSC strain B2-3, the hiPSC strain mB2-3, a hiPSC strain 648A1 (Reference Literature 3 below), and a human embryonic stem cell (hESC) strain H9 (Reference Literature 4 below) were used. These cell strains were cultured in the Essential 8 medium (manufactured by Thermo Fisher Scientific) free of feeder cells under the conditions of 37°C and 5% CO₂. The same applies to the cell culture conditions below unless otherwise stated. The hESC strain H9 was provided by the Ministry of Education, Culture, Sports, Science and Technology (MEXT, Japan).

Reference Literature 3: K. Okita, et al., "An efficient nonviral method to generate integration-free human-induced pluripotent stem cells from cord blood and peripheral blood cells", Stem Cells 31(3) (2013) 458-66.

Reference Literature 4: J.A. Thomson, et al., "Embryonic Stem Cell Lines Derived from Human Blastocysts", Science 282(5391) (1998) 1145-1147.

### (3) Method for Differentiation into Human iPS-Derived Lung Progenitor Cells

FIG. 1 shows the stages of differentiation of hPSC-derived lung progenitor cells for 21 days. As shown in FIG. 1, the PSC strain differentiates into NKX2-1⁺ lung progenitor cells in stages (Reference Literature 5 and 6). As shown in FIG. 1, in Example 1, each of the PSC strains was cultured and was allowed to differentiate in stages, and thus NKX2-1⁺ lung progenitor cells were obtained. Specifically, 1.2×10⁶ PSCs (free of feeder cells) and a medium (referred to collectively as a "culture" hereinafter) were seeded in a 6-well plate coated with Geltrex (registered trademark) (manufactured by Thermo Fisher Scientific). The composition of the medium corresponded to that of an RPMI medium (manufactured by Nacalai Tesque) containing 100-ng/ml Activine A (manufactured by Oriental Yeast), 1-µmol/l CHIR99021 (manufactured by Axon Medchem), 2% B27 supplement (manufactured by Thermo Fisher Scientific), and 50-U/ml penicillin-streptomycin (manufactured by Thermo Fisher Scientific). The medium was replaced with a new medium every other day.

Reference Literature 5: Y Yamamoto, et al., "Long-term expansion of alveolar stem cells derived from human iPS cells in organoids", Nat Methods 14(11) (2017) 1097-1106.

Reference Literature 6: Y Korogi, et al., "In Vitro Disease Modeling of Hermansky-Pudlak Syndrome Type 2 Using Human Induced Pluripotent Stem Cell-Derived Alveolar Organoids", Stem Cell Reports 12(3) (2019) 431-440.

On Day 0 of the culture, 10-µmol/l Y27632 (manufactured by LC laboratories) was added to the culture. Then, 0.25-mmol/l sodium butyrate (manufactured by FUJIFELM Wako Pure Chemical Corporation) was added to the culture on Day 1 of the culture, and 0.125-mmol/l sodium butyrate was added to the culture on Day 2 and Day 4 of the culture. Furthermore, on Day 6 to Day 10 of the culture, a Glutamax-supplemented DMEM/F12 basal medium (manufactured by Thermo Fisher Scientific) containing 2% B27 supplement, 50-U/ml penicillin-streptomycin, 0.05-mg/ml L-ascorbic acid (manufactured by FUJIFILM Wako Pure Chemical Corporation), and 0.4-mmol/l monothioglycerol (manufactured by FUJIFELM Wako Pure Chemical Corporation) supplemented with 100-ng/ml Noggin (manufactured by Proteintech) and 10-µmol/l SB431542 (manufactured by FUJIFILM Wako Pure Chemical Corporation) was used for culture of the PSC strains. Then, on Day 10 to Day 14 of the culture, the above-mentioned basal medium supplemented with 20-ng/ml BMP4 (manufactured by Proteintech), all-trans retinoic acid (manufactured by Sigma-Aldrich), and CHIR99021 was used for culture of the PSC strains. Note that the concentration of all-trans retinoic acid/CHIR99021 after the supplementation was set to the following concentration for each cell strain. Specifically, the concentration of all-trans retinoic acid/CHIR99021 after the supplementation was 0.5 µmol/l / 3.5 µmol/l for the hESC strain H9, 0.05 µmol/l / 3.0 µmol/l for the hiPSC strains B2-3 and mB2-3, and 1.0 µmol/l / 2.5 µmol/l for the hiPSC strain 648A1. On Day 14 to Day 21 of the culture, factors CHIR99021, FGF10 (manufactured by PeproTech), KGF (manufactured by PeproTech), and DAPT (manufactured by FUJIFILM Wako Pure Chemical Corporation) were added to the cultures of the PSC strains to concentrations of 3 µmol/l, 10 ng/ml, 10 ng/ml, and 20 µmol/l, respectively.

On Day 21 of the culture, CPM+ cells were separated using the method described in Reference Literature 7 below in which magnetic beads are used, and thus NKX2-1⁺ human lung progenitor cells (hLPs) were isolated. In the separation method above, a mouse anti-human CPM (manufactured by FUJIFILM Wako Pure Chemical Corporation) and anti-mouse IgG microbeads (manufactured by Miltenyi Biotec) were used.

Reference Literature 7: S. Konishi, et al., "Directed Induction of Functional Multi-ciliated Cells in Proximal Airway Epithelial Spheroids from Human Pluripotent Stem Cells", Stem Cell Reports 6(1) (2016) 18-25.

### (4) Preparation of Cell Microfiber (Structure of Example)

FIG. 2 shows proliferation culture and differentiation culture of hLPs in which a cell microfiber is used. As shown in FIG. 2, a structure (also referred to as a "cell microfiber" hereinafter) with a core-shell conformation was prepared in order to perform proliferation culture of hLPs in three-dimensional (3D) culture. A duplex concentric microfluidic device formed by combining an elongated glass capillary, a rectangular grass tube, and a self-prepared three-way connector was used to prepare the cell microfiber (see Reference Literature 8 and 9). As shown in FIG. 2, with the fluidic device, a first laminar flow (core stream) of a cell suspension is formed at the central portion, a second laminar flow (shell stream) of a pre-gel solution, which will be described later, can be formed on the outer circumference of the first laminar flow, and a third laminar flow (sheath stream) of an aqueous solution of calcium chloride can be formed on the outer circumference of the second laminar flow. Specifically, a pre-gel solution of a physiological saline supplemented with 1.0% I-1G sodium alginate (manufactured by KIMICA) was used for the shell stream of the cell microfiber. A mixed solution of 100-mmol/l calcium chloride (manufactured by Nacalai Tesque) and 3% sucrose (manufactured by Nacalai Tesque) was used for the sheath stream. The storage elastic modulus of the obtained cell microfiber measured using the above-described measurement method was about 900 Pa.

Reference Literature 8: H. Onoe, et al., "Metre-long cell-laden microfibres exhibit tissue morphologies and functions", Nat Mater 12(6) (2013) 584-90.

Reference Literature 9: K. Ikeda, et al., "Cell fiber-based three-dimensional culture system for highly efficient expansion of human induced pluripotent stem cells", Sci. Rep. 7(1) (2017)2850.

### (5) Proliferation Culture of hLPs Using Cell Microfiber

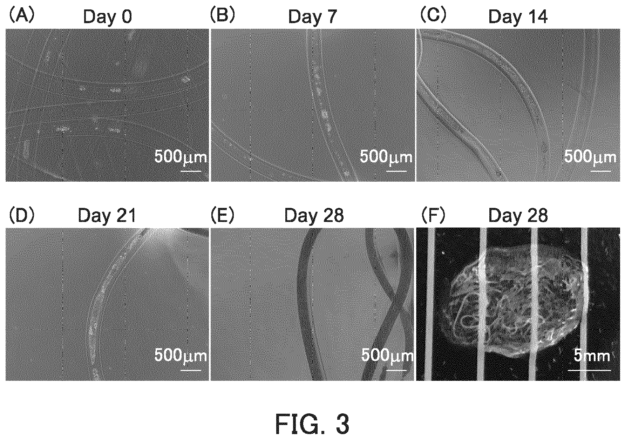

The hLPs isolated in Example 1(3) above were diluted to a concentration of 2.0×10⁵ cells/20 µl (1.0×10⁷ cells/ml) using a proliferation medium. The proliferation medium had a composition as follows: 3-µmol/l CHIR99021, 100-ng/ml FGF10, 10-ng/ml KGF, 20-µmol/l DAPT (manufactured by FUJIFILM Wako Pure Chemical Corporation), and 10-µmol/l Y-27632. The diluted hLPs were ejected into the core stream to prepare a cell microfiber that included the cells. In the preparation of the cell microfiber, the flow rates of the core stream, the shell stream, and the sheath stream were 50 µl/min, 200 µl/min, and 3.6 ml/min, respectively. After being prepared, the cell-capsule microfiber was subjected to culture in the proliferation medium as it is to perform proliferation culture. The proliferation medium was replaced with a new medium every other day. The proliferation culture was finished 4 weeks (28 days) after the start of the proliferation culture. In the proliferation culture, the cell microfiber was observed using an optical microscope when a predetermined period (0, 7, 14, 21, or 28 days) elapsed after the start of the culture. FIG. 3 shows the results of the observation on proliferation in the cell microfiber containing the hiPSC strain B2-3-derived hLP cells.

FIG. 3 shows photographs illustrating proliferation of hLPs in the cell microfibers. FIGS. 3(A) to 3(F) show the states of the cell microfibers on Day 0, Day 7, Day 14, Day 21, Day 28 and Day 28, respectively, after the start of the proliferation culture. Note that FIG. 3(F) is a photograph illustrating the entirety of a spherical cell aggregate formed through aggregation of the cell microfibers on Day 28 after the start of the proliferation culture. It was found that, as shown in FIGS. 3(A) to 3(F), after forming a cell mass in the cell microfiber, the hLPs proliferated in the longitudinal direction and finally formed a filamentous structure with a uniform diameter. Accordingly, it was confirmed that the hLPs proliferated in the cell microfiber.

### (6) Evaluation of Gene Expression in Proliferation Culture of hLPs

It has been reported that 3D cell culture has higher scalability than 2D cell culture (see Reference Literature 10 and 11). Meanwhile, in general, differentiation of cells progresses as proliferation of cells progresses. Therefore, differentiation markers, which will be described later, were used as indicators to examine if the cell quality could be maintained in 3D proliferation culture of hLPs in which the cell microfiber was used compared with 2D proliferation culture. The 3D proliferation culture of the hLPs in which the cell microfiber is used was performed in the same manner as in Example 1(4) and Example 1(5) above.

Reference Literature 10: M. Serra, et al., "Process engineering of human pluripotent stem cells for clinical application, Trends Biotechnol", 30(6) (2012) 350-9.

Reference Literature 11: Y Lei and D.V. Schaffer, "A fully defined and scalable 3D culture system for human pluripotent stem cell expansion and differentiation", Proc. Natl. Acad. Sci. U. S. A. 110(52) (2013) E5039-48.

A medium (2D cell medium) containing 3-µmol/l CHIR99021, 100-ng/ml FGF10, 10-ng/ml KGF, 20-µmol/l DAPT (FUJIFILM Wako Pure Chemical Corporation), and 10-µmol/l Y-27632 was used in 2D cell culture. The hLPs isolated in Example 1(3) above were used. 2.0×10⁵ hLPs were suspended in 1 ml of the 2D cell medium and were seeded in a 12-well cell culture plate. The hLPs were subcultured once after 2 weeks, and were collected using Accutase (manufactured by Innovative Cell Technologies) after another 2 weeks. The 2D cell medium was replaced with a new medium every other day.

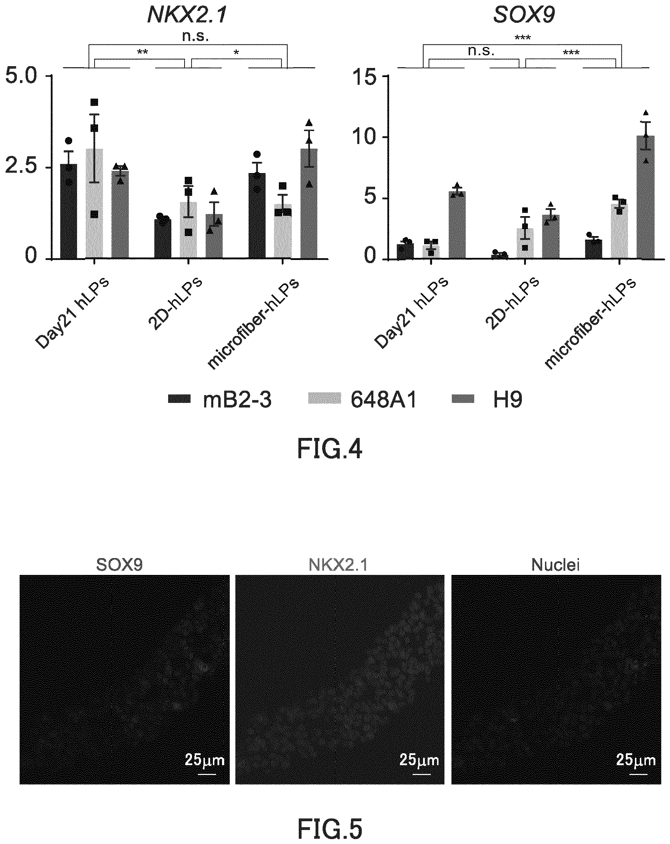

Differentiation markers, which will be described later, on cells after the 3D proliferation culture or the 2D proliferation culture were measured using quantitative reverse transcription PCR (qRT-PCR). Total RNA was extracted from the cells after the culture using PureLink RNA mini kit (manufactured by Thermo Fisher Scientific) or NucleoSpin RNA Plus XS (manufactured by Takara Bio Inc.). cDNA was synthesized using 80 ng of the obtained total RNA and SuperScript III Reverse Transcriptase (manufactured by Thermo Fisher Scientific). Then, a real-time PCR measurement apparatus QuantStudio 3 (manufactured by Applied Biosystems) was used to measure the gene expression levels of NKX2.1 (lung progenitor cell marker) and SOX9 (lung progenitor cell marker) in the reaction solution prepared using the cDNA, primer sets below, and Power SYBR Green PCR Mix (manufactured by Thermo Fisher Scientific). β-actin was used as an internal standard gene. The expression levels of the genes were compared with the gene expression levels in the 17th, 18th, and 22nd week in a human fetal lung (manufactured by Agilent Technologies, #540177, Lot. 0006055802). A control was carried out in the same manner, except that the hLPs prepared in Example 1(3) above were used. FIG. 4 shows the results. Note that a statistical analysis was carried out using software (GraphPad Prism 7 software, manufactured by GraphPad) (the same applies hereinafter).
- Primer set for NKX2.1
   Forward primer (Sequence ID No. 1)
      5' -AGGACACCATGAGGAACAGC-3'
   Reverse primer (Sequence ID No. 2)
      5'-GCCATGTTCTTGCTCACGTC-3'
- Primer set for SOX9
   Forward primer (Sequence ID No. 3)
      5'-GAGGAAGTCGGTGAAGAACG-3'
   Reverse primer (Sequence ID No. 4)
      5' -ATCGAAGGTCTCGATGTTGG-3'
- Primer set for β actin
   Forward primer (Sequence ID No. 5)
      5'-CAATGTGGCCGAGGACTTTG-3'
   Reverse primer (Sequence ID No. 6)
      5'-CATTCTCCTTAGAGAGAAGTGG-3'

FIG. 4 shows graphs illustrating the gene expression levels. In FIG. 4, the horizontal axes indicate the types of samples, and the vertical axes indicate the gene expression levels. As shown in FIG. 4, in the 2D cell culture (2D-hLPs), the expression levels of the lung progenitor cell markers (NKX2.1 and SOX9) decreased compared with the control group (Day 21 hLPs). Meanwhile, in the 3D cell culture (microfiber-hLPs), the expression levels of the lung progenitor cell markers were the same or increased compared with the control group. It was assumed from these results that culturing the hLPs in the cell microfiber allowed the hLPs to proliferate with the differentiation state being maintained, that is to say, as lung progenitor cells.

### (7) Evaluation of Protein Expression in Proliferation Culture of hLPs

It was examined if the expression of NKX2.1 protein and the expression of SOX9 protein in the hLPs after the 3D cell culture corresponded to the characteristics of human fetal lung progenitor cells described in Reference Literature 12 and 13. The hLPs were prepared in the same manner as in Example 1(3) to Example 1(5) above, except that human embryonic stem cells (hESC strain H9) were used instead of the hiPSC strain mB2-3, and the thus obtained hLPs were used to prepare a cell microfiber. Then, after proliferation culture was performed using the proliferation medium for 28 days, NKX2.1 (lung progenitor cell marker) and SOX9 (lung progenitor cell marker) in the obtained cell microfiber were immunostained. Specifically, the cell microfiber was fixed using PBS containing 4% paraformaldehyde at 4°C overnight. After the fixation, the cell microfiber was washed twice using a physiological saline, and was left to stand in 30% sucrose at 4°C overnight. After being left to stand, the cell microfiber was embedded in OCT Compound (manufactured by Sakura Finetek), was frozen at -80°C, and was then sliced to a thickness of 10 µm, and thus a sample was prepared. The obtained sample was permeabilized using PBS containing 0.2% Triton X-100 at room temperature (about 25°C, the same applies hereinafter) for 15 minutes. After the permeabilization, the sample was blocked using 1% BSA/PBS containing 5% Normal donkey serum (manufactured by EMD-Millipore) at room temperature for 30 minutes. After the blocking, the sample was reacted with a primary antibody at 4°C overnight, and was then stained using a secondary antibody at room temperature for 30 minutes. Hoechst 33342 (DOJINDO) was added to the secondary antibody solution to stain the nucleus. As the primary antibody, a mouse anti-TTF1 antibody (×500-fold, Cat.No: sc-53136, manufactured by Santa Cruz Biotechnology) was used to stain NKX2.1, and a rabbit anti-SOX9 antibody (×500 dilution, Cat.No: ab185230, manufactured by Abcam) was used to stain SOX9. As the secondary antibody, an Alexa Flour 488-labeled donkey anti-mouse IgG antibody (×500-fold, Cat.No: A-21202, manufactured by Thermo Fisher Scientific) and an Alexa Flour 546-labeled donkey anti-rabbit IgG antibody (×500-fold, Cat.No: A-10040, manufactured by Thermo Fisher Scientific) were used. After the staining, the sample was observed using a fluorescent microscope (BZ-X710 manufactured by Keyence, or TCS SP8 manufactured by Leica Microsystems). FIG. 5 shows the results.

Reference Literature 12: A.J. Miller, et al., "In Vitro Induction and In Vivo Engraftment of Lung Bud Tip Progenitor Cells Derived from Human Pluripotent Stem Cells", Stem Cell Reports 10(1) (2018) 101-119.

Reference Literature 13: M.Z. Nikolic, et al., "Human embryonic lung epithelial tips are multipotent progenitors that can be expanded in vitro as long-term self-renewing organoids", eLife 6 (2017) e26575.

FIG. 5 shows photographs illustrating immunostained images of the hLPs that proliferated in the cell microfibers. In FIG. 5, the photographs illustrate the immunostained images of the SOX9, the NKX2.1, and the nuclei in the order from left to right. As shown in FIG. 5, the majority of the hLPs expressed SOX9 and NKX2.1. These results corresponded to the characteristics of human fetal lung progenitor cells observed in Reference Literature 12 and 13 above. Also, it was found that the hLPs that had proliferated in the cell microfiber expressed SOX9 protein and NKX2.1 protein as in the case of the human fetal lung progenitor cells.

It was found from these results that, in the 3D proliferation culture of the hLPs in which the cell microfiber was used, the expression levels of the differentiation markers were the same as those before the proliferation culture compared with the 2D proliferation culture, that is, the differentiation state was maintained. Also, it was found that, in the 3D proliferation culture of the hLPs in which the cell microfiber was used, SOX9 protein and NKX2.1 protein were expressed as in the case of the human fetal lung progenitor cells. It was found from the results above that the 3D proliferation culture of the hLPs in which the cell microfiber is used is suitable for maintenance culture of the hLPs.

### (8) Evaluation of Ability of hLPs after Proliferation to Differentiate into Alveolar Epithelial Cells

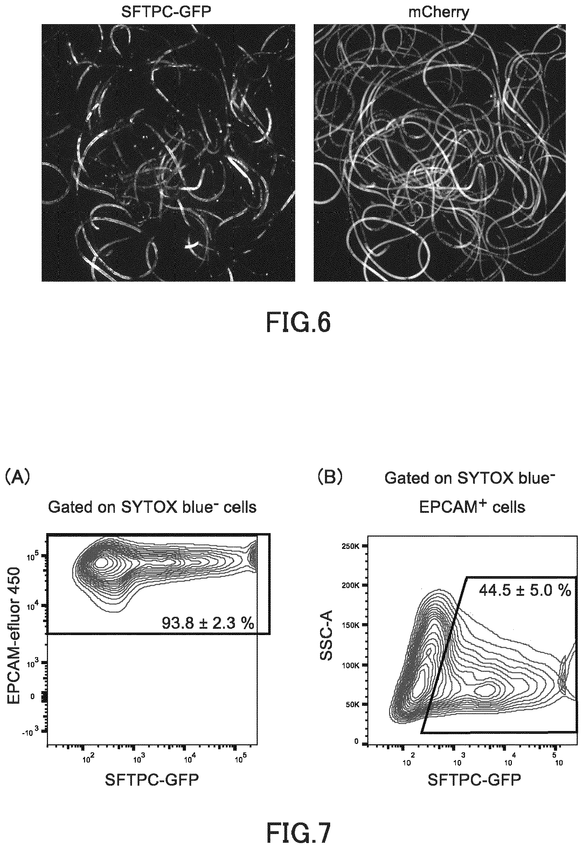

In order to check whether the hLPs (microfiber hLPs) cultured in the cell microfiber retain the differentiation ability, differentiation of the cell microfiber hLPs after the culture into alveolar cells was evaluated. First, differentiation of the hLPs that had proliferated in the cell microfiber into alveolar cells was induced. Specifically, after the stage of proliferation of the cell microfiber obtained in the same manner as in Example 1(3) to Example 1(5) above was finished, the cell microfiber was washed once using a physiological saline. After the washing, the medium for the cell microfiber was replaced with an alveolus differentiation medium. As the alveolus differentiation medium, a Ham's F12 medium (manufactured by FUJIFILM Wako Pure Chemical Corporation) supplemented with the following factors was used: 50-nmol/l dexamethasone (manufactured by Sigma-Aldrich), 100-µmol/l 8-Br-cAMP (manufactured by Biolog), 100-µmol/l 3-isobutyl-1-methylxanthine (manufactured by FUJIFILM Wako Pure Chemical Corporation), 10-ng/ml KGF (manufactured by PeproTech), 1% B-27 supplement (manufactured by Thermo Fisher Scientific), 0.25% BSA (manufactured by Thermo Fisher Scientific), 15-mmol/l HEPES (manufactured by Sigma-Aldrich), 0.8-mmol/l calcium chloride (manufactured by FUJIFILM Wako Pure Chemical Corporation), 0.1% ITS premix (manufactured by Corning), 50-U/ml penicillin-streptomycin, 3-µmol/l CHIR99021, 10-µmol/l SB431542, and 10-µmol/l Y27632 (see Reference Literature 5 above). In the differentiation induction, the cell microfiber was cultured for 14 days. The medium was replaced with a new medium every other day. In the differentiation induction, fibroblast feeder cells were not added. After the differentiation induction, live imaging was performed using a fluorescent microscope (BZ-X710 manufactured by Keyence). FIG. 6 shows the results.

FIG. 6 shows the results obtained through live imaging observation on differentiation of the hLPs in the cell microfibers into alveolar epithelial cells. Cells positive for the type-II alveolar epithelial cell marker SFTPC-GFP were observed. It was found from the results above that the hLPs that had proliferated in the cell microfiber could differentiate into alveolar epithelial cells.

In order to evaluate differentiation of the hLPs in the cell microfiber into alveolar epithelial cells from another viewpoint, flow cytometry was used. Specifically, cells were isolated from the cell microfiber after the proliferation, and then a single cell suspension was prepared. The single cell suspension was prepared as follows. First, the cell microfiber hLPs that had proliferated were washed using D-PBS(-), and were then cultured in 0.5-mmol/l EDTA/PBS at room temperature for 10 minutes. Next, after washed, the cell microfiber was treated with Accumax (Innovative Cell Technologies) at 37°C for 10 minutes.

Next, the single cell suspension was stained using a primary antibody at 4°C for 20 minutes. After the primary antibody staining, the single cell suspension was washed twice and was then stained using a secondary antibody at 4°C for 20 minutes. After the staining, the cells were washed twice and were then stained through dead cell staining. 1-µmol/l propidium iodide (manufactured by Nacalai Tesque) or 1-µmol/l SYTOX Blue Dead Cell Stain (manufactured by Thermo Fisher Scientific) was used for the dead cell staining. A fluorescence-activated cell sorting (FACS) analysis was performed using FACSAria III (manufactured by BD Biosciences). An anti-EPCAM antibody (Cat.No: sc-66020, manufactured by Santa Cruz Biotechnology) was used as the primary antibody, and an eFlour 450-labeled anti-mouse IgG antibody (Cat.No: 48-4015-82, manufactured by Thermo Fisher Scientific) was used as the secondary antibody. FIG. 7 shows the results.

FIG. 7 shows graphs illustrating the results of flow cytometry. FIG. 7(A) shows the results obtained when gating of cells positive for SYTOX Blue in the dead cell staining was performed, and FIG. 7(B) shows the results obtained when gating of EPCAM positive cells was performed in FIG. 7(A). In FIG. 7(A), the vertical axis indicates the fluorescence intensity of EPCAM (epithelial cell marker), and the horizontal axis indicates the fluorescence intensity of SFTPC (type-II alveolar epithelial cell marker). In FIG. 7(B), the vertical axis indicates the intensity of SSC-A (side scattered light signal), and the horizontal axis indicates the fluorescence intensity of SFTPC (type-II alveolar epithelial cell marker). As shown in FIGS. 7(A) and 7(B), it could be confirmed that about half of the hLPs that had proliferated in the cell microfiber differentiated from lung progenitor cells into alveolar epithelial cells. It was found from the results above that the hLPs that had proliferated had an ability to differentiate into an alveolar epithelial cell.

### (9) Frequency of SFTPC-GFP Positive hLP-Derived Alveolar Epithelial Cells after Proliferation

The rate of SFTPC-GFP positive cells was examined using flow cytometry by comparing alveolar epithelial cells that had differentiated from the cell microfiber hLPs and fibroblast-free alveolar organoids described in Reference Literature 5 above.

The fibroblast-free alveolar organoids were produced using the method described in Reference Literature 5 above. Specifically, 2.0×10⁵ hLPs isolated in Example 1(3) were suspended in 200 µl of a medium and were seeded in a plate with a non-adhesive surface. The medium was the same as the alveolus differentiation medium used in Example 1(8) above. Two days after the seeding, cell masses were collected and centrifuged. Pellet obtained through the centrifugation was resuspended in 20 µl of a growth factor reduced Matrigel (manufactured by Corning), and 1 ml of the alveolus differentiation medium was added thereto. Then, the cells were cultured until Day 7 of the culture in a 12-well cell culture plate. After the culture, the fibroblast-free alveolar organoids were separated using 0.1% trypsin/EDTA and collected.

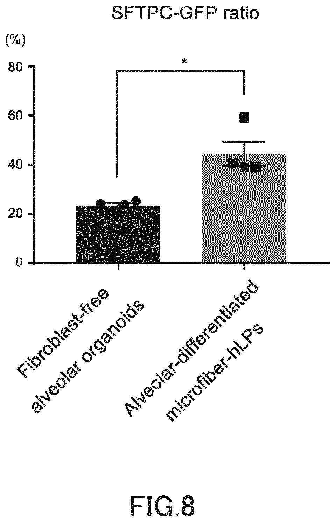

Flow cytometry was performed using a single cell suspension derived from the cell microfiber prepared in the same manner as in Example 1(8) above and a single cell suspension formed using the fibroblast-free alveolar organoids. The preparation of the single cell suspension and the flow cytometry were performed in the same manner as in Example 1(8) above. Then, the rate of GFP positive cells in each sample was calculated. Note that the hiPSC strain B2-3 was used as the pluripotent stem cell strain instead of the hiPSC strain mB2-3. FIG. 8 shows the results.

FIG. 8 is a graph illustrating the calculated rates of SFTPC-GFP positive cells. In FIG. 8, the vertical axis indicates the frequency, and the horizontal axis indicates the types of cells. The rate of SFTPC-GFP positive cells in the alveolar epithelial cells in the cell microfiber was approximately twice as high as that in the fibroblast-free alveolar organoids. That is to say, the hLPs that had proliferated in the cell microfiber differentiated into alveolar epithelial cells at higher efficiency.

### (10) Evaluation of Gene Expression in Alveolar Epithelial Cells

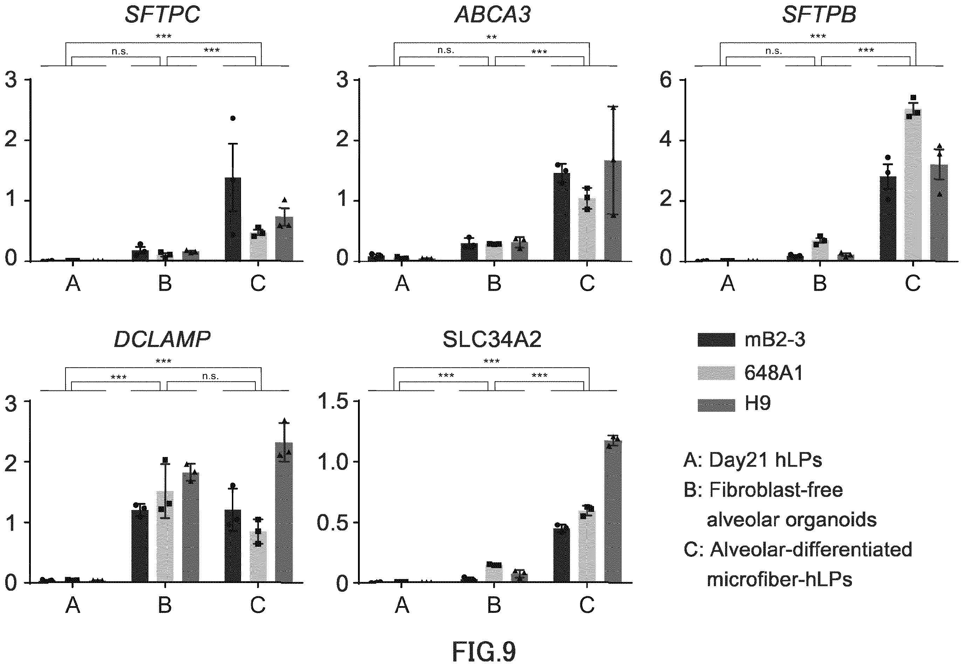

The gene expression in the alveolar epithelial cells in the cell microfiber prepared in Example 1(8) was examined using qRT-PCR. The gene expression levels were measured by performing the RT-PCR in the same manner as in Example 1(6), except that primer sets for type-II alveolar epithelial cell markers, which will be described later, were used instead of the primer sets for lung progenitor cell markers. The target type-II alveolar epithelial cell markers were SFTPC, ABCA3, SFTPB, DCLAMP, and SLC34A2. Primer sets for these genes were as follows. In a control 1, the gene expression level was measured in the same manner, except that hLPs (Day 21 hLPs) prior to culture in the cell microfiber were used. In a control 2, the gene expression level was measured in the same manner, except that the fibroblast-free alveolar organoids were used. β actin was used as an internal standard gene as in Example 1(6) above. FIG. 9 shows the results.
- Primer set for SFTPC
   Forward primer (Sequence ID No. 7)
      5'-GCAAAGAGGTCCTGATGGAG-3'
   Reverse primer (Sequence ID No. 8)
      5'-TGTTTCTGGCTCATGTGGAG-3'
- Primer set for ABCA3
   Forward primer (Sequence ID No. 9)
      5' - TCTCCTTCAGCTTCATGGTCAG-3'
   Reverse primer (Sequence ID No. 10)
      5'-TGGCTCAGAGTCATCCAGTTG-3'
- Primer set for SFTPB
   Forward primer (Sequence ID No. 11)
      5'-GAGCCGATGACCTATGCCAAG-3'
   Reverse primer (Sequence ID No. 12)
      5' -AGCAGCTTCAAGGGGAGGA-3'
- Primer set for DCLAMP
   Forward primer (Sequence ID No. 13)
      5'-ACCGATGTCCAACTTCAAGC-3'
   Reverse primer (Sequence ID No. 14)
      5' - TGACACCTTAGGCGGATTTT-3'
- Primer set for SLC34A2
   Forward primer (Sequence ID No. 15)
      5'-TCGCCACTGTCATCAAGAAG-3'
   Reverse primer (Sequence ID No. 16)
      5'-CTCTGTACGATGAAGGTCATGC-3'

FIG. 9 shows graphs illustrating the gene expression levels. In FIG. 9, the horizontal axes indicate the types of samples, and the vertical axes indicate the gene expression levels. As shown in FIG. 9, the gene expression levels of the type-II alveolar epithelial cell markers in the alveolar epithelial cells in the cell microfiber increased compared with the Day 21 hLPs. Also, the gene expression levels of the type-II alveolar epithelial cell markers increased irrespective of the type of human pluripotent stem cell strain. Furthermore, it was found that the gene expression levels of the type-II alveolar epithelial cell markers in the alveolar epithelial cells in the cell microfiber were higher than those in the cell-free alveolar organoids.

### (11) Evaluation of Protein Expression in Alveolar Epithelial Cells

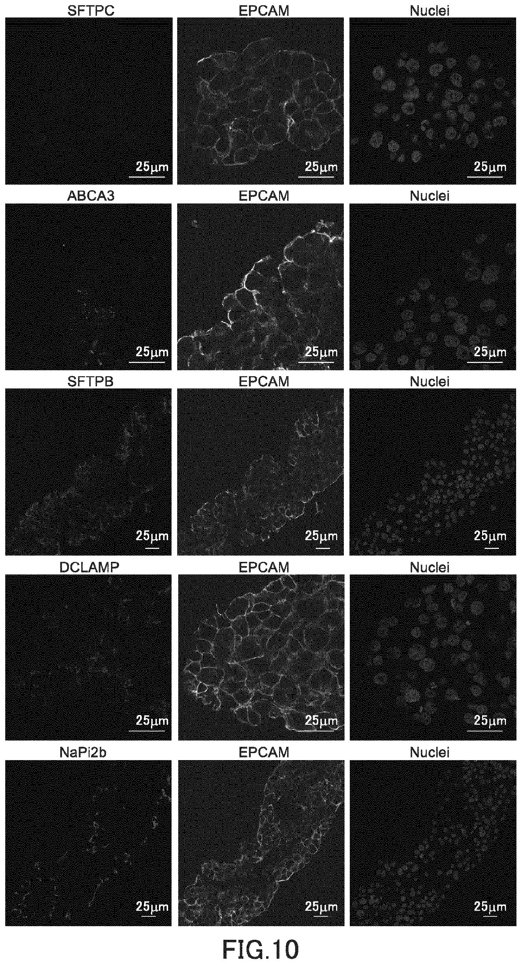

It was evaluated if the alveolar epithelial cells in the cell microfiber prepared in Example 1(8) above expressed proteins expressed in type-II alveolar epithelial cells. To evaluate the protein expression, alveolar epithelial cells induced from human embryonic stem cells (hESC strain H9) in the cell microfiber were used and immunostained. The immunostaining was performed in the same manner as in Example 1(7), except that primary antibodies and secondary antibodies below were used, and the cells were permeabilized using methanol in the case of ABCA staining. As the primary antibody, a mouse anti-SFTPC antibody (×100-fold dilution, Cat.No: sc-13979, manufactured by Santa Cruz Biotechnology) was used to stain SFTPC, a goat anti-EPCAM antibody (×100-fold dilution, Cat.No: AF960, manufactured by R&D systems) was used to stain EPCAM, a mouse anti-ABCA3 antibody (clone name: ×100-fold dilution, Cat.No: WMAB-ABCA3-17, manufactured by Seven Hills) was used to stain ABCA3, a rabbit anti-SFTPB antibody (×100-fold dilution, Cat.No: AB3430, manufactured by EMD-Millipore) was used to stain SFTPB, a mouse anti-DCLAMP antibody (×100-fold dilution, Cat.No: IM3448, manufactured by Beckman Coulter) was used to stain DCLAMP, and a mouse anti-NaPi2b antibody (provided by Dr. Gerd Ritter, clone name MX35 (see Reference Literature 13) was used to stain NaPi2b. As the secondary antibody, an Alexa Fluor 546-labeled donkey anti-mouse IgG (H + L) (Cat.No: A-10036, manufactured by Thermo Fisher Scientific), an Alexa Fluor 546-labeled donkey anti-rabbit IgG (H + L) antibody (Cat.No: A-10040, manufactured by Thermo Fisher Scientific), or an Alexa Fluor 488-labeled anti-goat IgG (H + L) antibody (Cat.No: A-11055, manufactured by Thermo Fisher Scientific) was reacted. Hoechst 33342 (DOJINDO) was added to the secondary antibody solution to stain the nucleus. After the staining, the sample was observed using a fluorescent microscope (BZ-X710 manufactured by Keyence, TCS SP8 manufactured by Leica Microsystems). FIG. 10 shows the results.

Reference Literature 13: Beatrice W. T. Yin et al., "Monoclonal antibody MX35 detects the membrane transporter NaPi2b (SLC34A2) in human carcinomas", Cancer Immun., 2008 Feb 6;8: p3.

FIG. 10 shows photographs illustrating immunostained images of alveolar epithelial cells in the cell microfibers. As shown in FIG. 10, the alveolar epithelial cells in the cell microfiber expressed the type-II alveolar epithelial cell marker (SFTPC, ABCA3, SFTPB, DCLAMP, or SLC34A2), which is shown in gray, within the region surrounded by EPCAM, which is shown in white. As revealed in these results, the cells in the cell microfiber expressed the epithelial cell markers, specifically the type-II alveolar epithelial cell markers, and it was thus found that the cells had differentiated into type-II alveolar epithelial cells. That is to say, it was confirmed that the hLPs that had proliferated in the cell microfiber had an ability to differentiate into an alveolar epithelial cell.

### (12) Observation of Lamellar Bodies of Alveolar Epithelial Cells

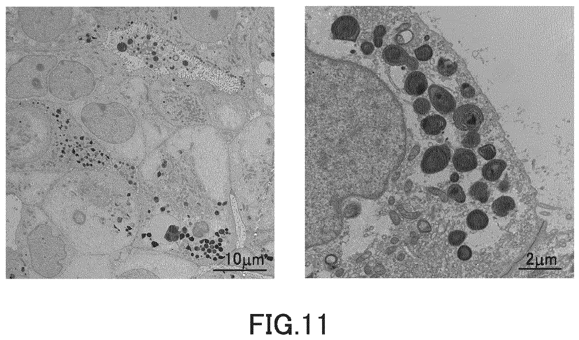

It was examined if the alveolar epithelial cells in the cell microfiber possess an intracellular organella specific to the type-II alveolar epithelial cells. A transmission electron microscope (TEM) was used to measure the intracellular organella specific to the type-II alveolar epithelial cells. Specifically, the cell microfiber was embedded in iPGell (manufactured by GenoStaft) in accordance with the protocol to prepare a sample. The sample was fixed using a fixative solution at 4°C for 2 hours. The composition of the fixative solution was as follows: 2.5% glutaraldehyde, 2% paraformaldehyde, 2% osmium oxide (VIII), 0.1% picric acid, 4% sucrose, and 0.1-mol/l phosphate buffer (pH 7.4). After the fixation, block staining was performed using 1% uranyl acetate (VI) at room temperature for 1 hour in the same manner as in Reference Literature 1 above. The obtained sample was embedded in Epon 812 for dehydration. After ultra-thin sections were prepared from the embedded sample, the obtained ultra-thin sections were stained using uranyl acetate and lead citrate and were then observed using an H-7650 transmission electron microscope (manufactured by Hitachi, Ltd.). FIG. 11 shows the results.

FIG. 11 shows photographs illustrating electron microscopic images of alveolar epithelial cells in the cell microfibers. As shown in FIG. 11, it was found that many lamellar bodies (the lamellar body is an intracellular organella specific to the type-II alveolar epithelial cells) were present in the alveolar epithelial cells in the cell microfiber. As revealed from the results above, the cells in the cell microfiber differentiated into alveolar epithelial cells, and it was thus confirmed that the hLPs that had proliferated in the cell microfiber had an ability to differentiate into an alveolar epithelial cell.

### (13) Differentiation into Airway Epithelial Cells

Differentiation of the hLPs cultured using the cell microfiber (microfiber hLPs) into airway cells was evaluated. First, differentiation of the hLPs that had proliferated in the cell microfiber into airway cells was induced. Specifically, after the stage of proliferation of the cell microfiber obtained in the same manner as in Example 1(3) to Example 1(5) above was finished, the cell microfiber was washed once using a physiological saline. After the washing, the medium for the microfiber was replaced with an airway differentiation medium. As the airway differentiation medium, a PneumaCult-ALI basal medium (manufactured by STEMCELL Technologies) supplemented with the following factors was used: PneumaCult-ALI Supplement, PneumaCult-ALI Maintenance Supplement, 4-µg/ml heparin, 1-µmol/l hydrocortisone, 10-µmol/l DAPT (manufactured by FUJIFILM Wako Pure Chemical Corporation), and 10-µmol/l Y-27632 (see Reference Literature 7 above). In the airway differentiation stage, the cell microfiber was cultured for 28 days. The medium was replaced with a new medium every other day.

### (14) Evaluation of Gene Expression in Airway Epithelial Cells

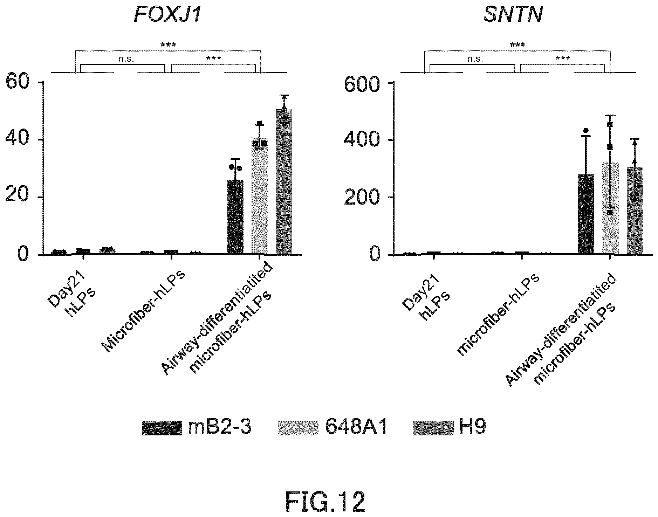

Next, the gene expression in the airway cells in the cell microfiber was examined using qRT-PCR. The gene expression levels were measured by performing the qRT-PCR in the same manner as in Example 1(6), except that primer sets for airway epithelium markers, which will be described later, were used instead of the primer sets for lung progenitor cell markers. The target airway epithelium markers were FOXJ1 and SNTN. Primer sets for these genes were as follows. In a control 1, the gene expression level was measured in the same manner, except that hLPs (Day 21 hLPs) prior to culture in the cell microfiber were used. In a control 2, the gene expression level was measured in the same manner, except that hLPs (microfiber hLPs) that had proliferated but had not been subjected to induction of differentiation into airway cells were used. β actin was used as an internal standard gene as in Example 1(6) above. FIG. 12 shows the results.
- Primer set for FOXJ1
   Forward primer (Sequence ID No. 17)
      5'-CCTGTCGGCCATCTACAAGT-3'
   Reverse primer (Sequence ID No. 18)
      5' -AGACAGGTTGTGGCGGATT-3'
- Primer set for SNTN
   Forward primer (Sequence ID No. 19)
      5'-GCTGCAAACCCAATTTAGGA-3'
   Reverse primer (Sequence ID No. 20)
      5'-TGCTCATCAAGTTCAGAAAGGA-3'

FIG. 12 shows graphs illustrating the gene expression levels. In FIG. 12, the horizontal axes indicate the types of samples, and the vertical axes indicate the expression levels of the genes. As shown in FIG. 12, the gene expression levels of the airway epithelial cell markers in the airway cells in the cell microfiber increased compared with the Day 21 hLPs. Also, the gene expression levels of the airway epithelial cell markers increased irrespective of the type of human pluripotent stem cell. Furthermore, it was found that the gene expression of the airway epithelial cell markers in the airway cells in the cell microfiber was higher than those in the microfiber-hLPs. It was found from the results above that the hLPs that had proliferated differentiated into airway epithelial cells.

### (15) Evaluation of Protein Expression in Airway Epithelial Cells

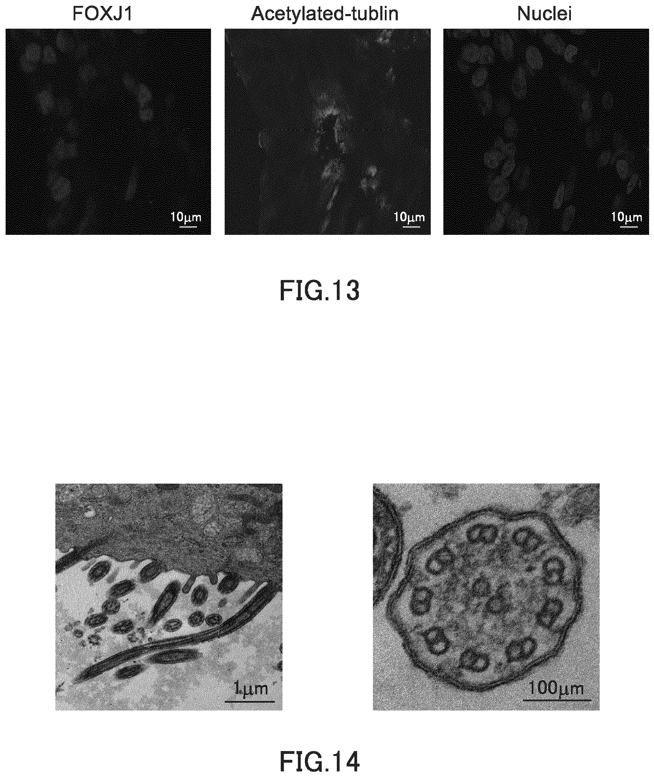

It was evaluated if the airway epithelial cells in the cell microfiber expressed proteins expressed in airway epithelial cells. To evaluate the protein expression, airway epithelial cells induced from human embryonic stem cells (hESC strain H9) in the cell microfiber were used and immunostained. The immunostaining was performed in the same manner as in Example 1(7). To stain FOXJ1, a mouse anti-FOXJ1 antibody (×500-fold dilution, Cat.No: 14-9965-82, manufactured by eBioscience) was used as the primary antibody, and an Alexa Fluor 488-labeled donkey anti-mouse IgG2b antibody (Cat.No: A-21141, manufactured by Thermo Fisher Scientific) was used as the secondary antibody. To stain acetylated-tubulin, a mouse anti-acetylated-tubulin antibody (×4000-fold dilution, Cat.No: T7451, manufactured by Sigma-Aldrich) was used as the primary antibody, and a Cy3-labeled donkey anti-mouse IgG1 antibody (Cat.No: 115-165-205, manufactured by Jackson Immunoresearch) was used as the secondary antibody. Hoechst 33342 (DOJINDO) was added to the secondary antibody solution to stain the nucleus. After the staining, the sample was observed using a fluorescent microscope (BZ-X710 manufactured by Keyence, TCS SP8 manufactured by Leica Microsystems). FIG. 13 shows the results.

FIG. 13 shows photographs illustrating immunostained images of airway epithelial cells in the cell microfibers. As shown in FIG. 13, the airway epithelial cells in the cell microfiber expressed the airway epithelial cell marker (FOXJ1 and acetylated tubulin). As revealed in these results, the cells in the cell microfiber expressed the airway epithelial cell markers, and it was thus found that the cells had differentiated into airway epithelial cells. That is to say, it was confirmed that the hLPs that had proliferated in the cell microfiber had an ability to differentiate into an airway epithelial cell.

### (16) Cilia Formation of Airway Epithelial Cells

It was evaluated if the airway epithelial cells in the cell microfiber had cilia, which can be found on airway epithelial cells. The cilia were observed using the transmission electron microscope (TEM) in the same manner as in Example 1(12). FIG. 14 shows the results.

FIG. 14 shows photographs illustrating electron microscopic images of airway epithelial cells in the cell microfibers. As shown in FIG. 14, it was found that many cilia were present on the cells in the cell microfiber. Also, ciliary motion was visually observed. As revealed from the results above, the cells in the cell microfiber differentiated into airway epithelial cells, and it was thus confirmed that the hLPs that had proliferated in the cell microfiber had an ability to differentiate into an airway epithelial cell. The hLPs that had proliferated in the cell microfiber had an ability to differentiate into an alveolar epithelial cell and an airway epithelial cell, and thus their differentiation ability is the same as that of lung progenitor cells. Accordingly, it was assumed that the hLPs that had proliferated in the cell microfiber proliferated while retaining the differentiation ability.

### (17) Gene Expression Profiling of Cell Microfiber hLPs

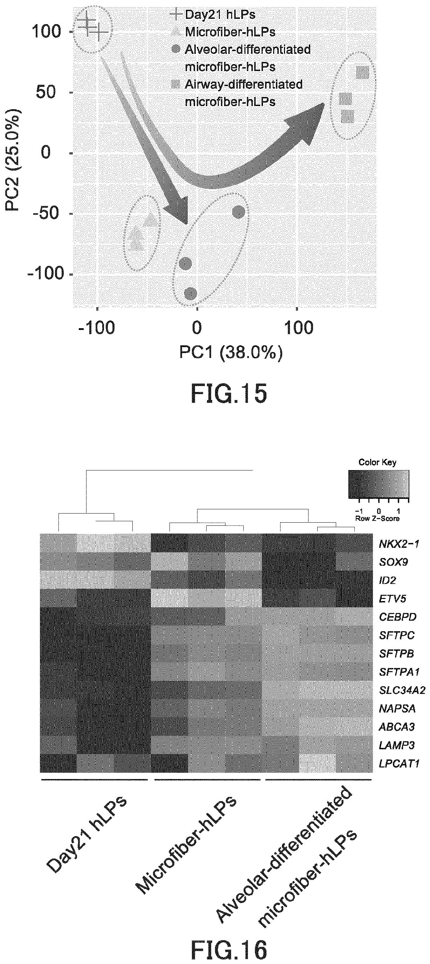

An RNA-seq analysis was conducted to perform gene expression profiling of the cell microfiber hLPs. In the RNA-seq analysis, samples prepared from hLPs (Day 21 hLPs) before included in the cell microfiber, hLPs (microfiber-hLPs) in the cell microfiber obtained in the same manner as in Example 1(5) above, alveolar epithelial cells (Alveolar-differentiated microfiber-hLPs) in the cell microfiber obtained in the same manner as in Example 1(8) above, and airway epithelial cells (Airway-differentiated microfiber-hLPs) in the cell microfiber obtained in the same manner as in Example 1(13) were used. Specifically, first, total RNA was extracted from each type of cells, and then the obtained total RNA and SMART-seq v4 Ultra Low Input RNA Kit (manufactured by Takara Bio Inc.) were used to synthesize and amplify single-stranded cDNA. A sequence library was prepared using the single-stranded cDNA and Nextera XT DNA Library Prep Kit (manufactured by Illumina). Sequencing of the sequence library was conducted based on the 100-bp pair-end method using a NovaSeq 6000 sequencer (manufactured by Illumina). The raw data of sequence reads were subjected to trimming of the adapter sequence using Trimmomatic, and the trimmed data were mapped in a human reference genome (GRCh37/hg19) using HISAT2. After the mapping, transcript assembly was performed using StringTie, and the gene expression levels of genes were calculated based on the read counts. iDEP.91 was used to analyze the RNA-seq data (see Reference Literature 15). In the RNA-seq analysis, cells derived from the hiPSC strain mB2-3 were used. FIGS. 15 to 19 show the results of the RNA-seq analysis.

Reference Literature 15: S.X. Ge, et al., "an integrated web application for differential expression and pathway analysis of RNA-Seq data", BMC Bioinformatics 19(1) (2018) 534.

FIG. 15 is a graph illustrating the results of a principal component analysis (PCA) performed on the RNA-seq data. In FIG. 15, the horizontal axis indicates a first principal component, and the vertical axis indicates a second principal component. As shown in FIG. 15, it was found that the hLPs that had proliferated in the cell microfiber (indicated by triangles) exhibited a gene expression profile closer to that of the alveolar epithelial cells in the cell microfiber (indicated by circles) than that of Day 21 hLPs (indicated by crosses).

FIG. 16 is a heat map illustrating comparison of the expression of genes relating to peripheral lung progenitor cells and type-II alveolar epithelial cells between the Day 21 hLPs, the hLPs that proliferated in the cell microfiber, and the alveolar epithelial cells in the cell microfiber. FIG. 16 shows the results from the clusters of the Day 21 hLPs, the hLPs that proliferated in the cell microfiber, and the alveolar epithelial cells in the cell microfiber in the order from left to right. In FIG. 16, target genes were shown on the right side of the heat map. As shown in FIG. 16, after the differentiation into the alveolar epithelial cell, the expression of the progenitor cell markers (NKX2-1 (NKX2.1), SOX9, ID2) decreased, whereas the expression of the type-II alveolar epithelial cell markers (CEBPD, SFTPC, SFTPB, SFTPA1, SLC34A2, NAPSA, ABCA3, LAMP3, LPCAT1) increased. The result of the gene expression pattern in the hLPs that had proliferated in the cell microfiber was intermediate between the results of the gene expression patterns in the Day 21 hLPs and the type-II alveolar epithelial cells.

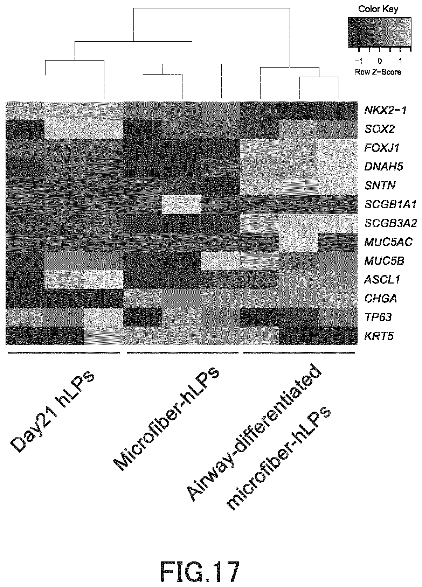

Next, FIG. 17 is a heat map illustrating comparison of the expression of genes relating to peripheral lung progenitor cells and airway epithelial cell markers between the Day 21 hLPs, the hLPs that proliferated in the cell microfiber, and the airway epithelial cells in the cell microfiber. FIG. 17 shows the results from the clusters of the Day 21 hLPs, the hLPs that proliferated in the cell microfiber, and the airway epithelial cells in the cell microfiber in the order from left to right. As shown in FIG. 17, after the differentiation into the airway epithelial cell, the expression of the progenitor cell markers (NKX2-1 (NKX2.1), SOX9) decreased, whereas the expression of the ciliated epithelial cell markers (FOXJ1, DNAH5, SNTN, SCGB1A1, SCGB3A2, MUC5AC, MUC5B, ASCL1, CHGA, TP63, KRT5) increased. The result of the gene expression pattern in the hLPs that had proliferated in the cell microfiber was intermediate between the results of the gene expression patterns in the Day 21 hLPs and the airway alveolar epithelial cells.

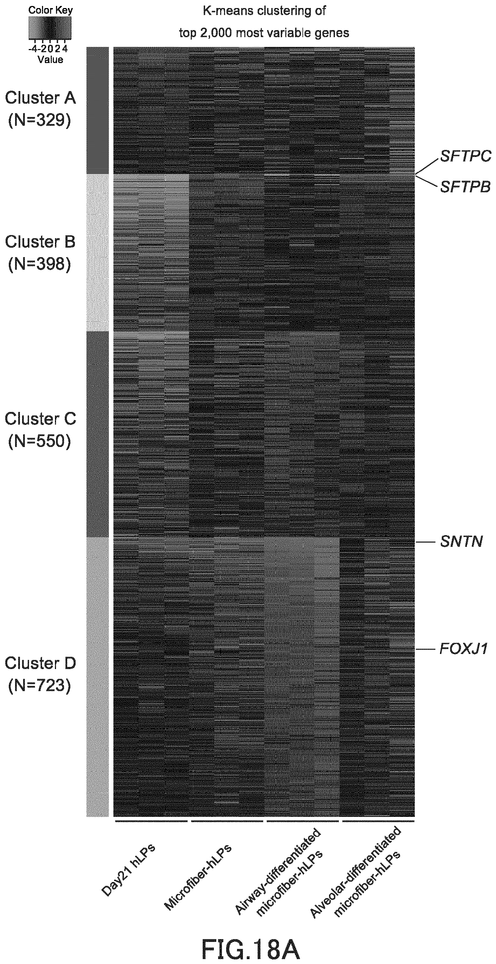

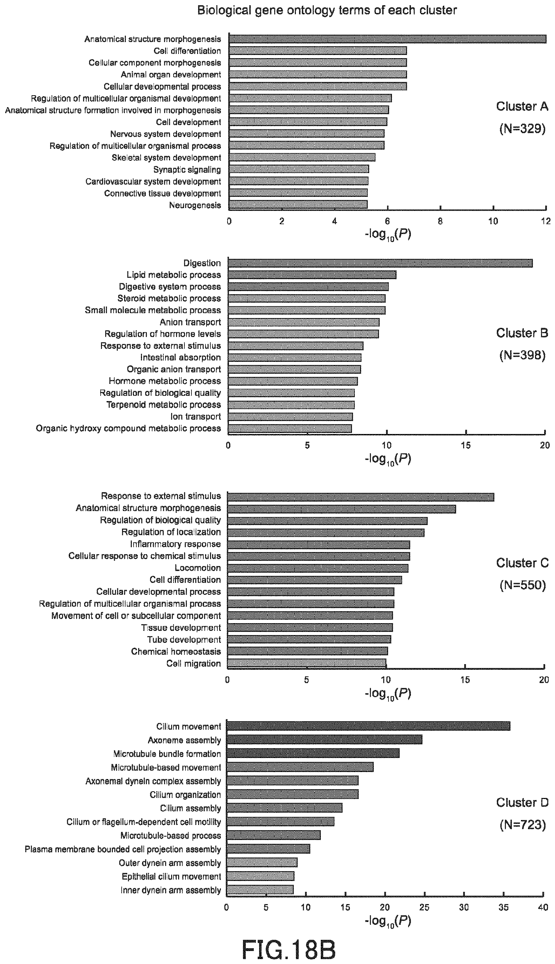

FIGS. 18A and 18B shows the results of a nonhierarchical cluster analysis based on the K-means method. FIG. 18A is a heat map illustrating the results of gene clusters of top-2000 genes that significantly varied between samples, and FIG. 18B shows graphs illustrating the results of an enrichment analysis performed on the clusters. In FIG. 18A, the vertical axis indicates the clusters, and the horizontal axis indicates the types of cells. In FIG. 18B, the vertical axis indicates the biological gene ontology, and the horizontal axis indicates the types of cells. As shown in FIGS. 18A and 18B, the expression of the cluster A significantly varied in the Day 21 hLPs, the expression of the cluster B significantly varied in the type-II alveolar epithelial cells in the cell microfiber, and the expression of the cluster C significantly varied in the airway epithelial cells in the cell microfiber. It was found from these results that the type-II alveolar epithelial cells in the cell microfiber and the airway epithelial cells in the cell microfiber could be distinguished from each other based on the cluster with significant expression variation. Meanwhile, the hLPs that had proliferated in the cell microfiber showed an expression variation pattern intermediate between those of the type-II alveolar epithelial cells and the airway epithelial cells.

### (18) Proliferation of hLPs

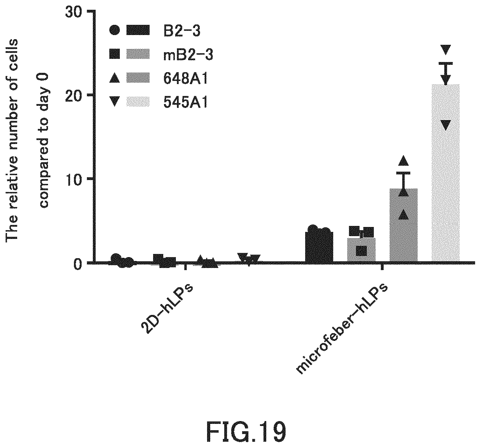

The hiPSC strain B2-3, the hiPSC strain mB2-3, the hiPSC strain 648A1, and the hiPSC strain 585A1 (see Reference Literature 3 above) were used as human pluripotent stem cells, and the degrees of cell proliferation were compared. Cell microfibers that included lung progenitor cells were prepared in the same manner as in Example 1(2) to Example 1(5) and then the proliferation culture was performed, except that cell microfibers were prepared using the hiPSC strain B2-3, the hiPSC strain mB2-3, the hiPSC strain 648A1, and the hiPSC strain 585A1 (see Reference Literature 3) as the human pluripotent stem cells and the concentrations of the hiPSC strain B2-3 and the hiPSC strain mB2-3 were set to 2.2×10⁵ cells/20 µl (1.1×10⁷ cells/ml). After the proliferation culture, the cells were collected from each of the cell microfibers, and the number of cells was counted. Then, the relative number of cells was calculated using the number of cells before the proliferation culture as a standard (1). Also, 2D cell culture was performed in the same manner as in Example 1(6) and the relative numbers of cells were calculated, except that lung progenitor cells prepared from the hiPSC strain B2-3, the hiPSC strain mB2-3, the hiPSC strain 648A1, and the hiPSC strain 585A1 as the lung progenitor cells. FIG. 19 shows the results.

FIG. 19 is a graph illustrating the relative numbers of cells after the proliferation culture. In FIG. 19, the horizontal axis indicates the types of culture, and the vertical axis indicates the relative numbers of cells. As shown in FIG. 19, in the 2D proliferation culture (2D-hLPs), the hLPs derived from any of the hiPS strains hardly proliferated. Meanwhile, in the case where the proliferation culture was performed in the cell microfiber, the hLPs derived from any of the hiPS strains proliferated.

It was found from the results above that the proliferation culture of the hLPs in which the cell microfiber was used was very useful as a method for increasing the number of cells while the cells retained an ability to differentiate into an alveolar epithelial cell or an airway epithelial cell. As described above, with 2D proliferation culture, human lung progenitor cells were difficult to proliferate while retaining the lung progenitor cell properties. However, it was found that using the structure of the present invention made it possible to perform proliferation culture of lung progenitor cells while allowing the differentiation ability to be retained.

### Example 2

It was checked whether lung progenitor cells proliferated in the cell microfiber in various culture conditions.

In the case where the hLPs proliferate and are then induced to type-II alveolar epithelial cells, the rate of the type-II alveolar epithelial cells in the obtained cell population increases. Accordingly, the proliferation was evaluated using the efficiency of differentiation into a type-II alveolar epithelial cell as an indicator.

### (1) Condition 1

The hLPs isolated in Example 1(3) above were diluted to 5.0×10⁷ cells/ml using the same proliferation medium as that in Example 1(5) above. Note that cells derived from the pluripotent stem cell hiPSC strain B2-3 were used as the hLPs. A cell microfiber that included the diluted cells was prepared in the same manner as in Example 1(5) above. The obtained cell microfiber was cultured in the proliferation medium for 4 weeks. The culture was carried out under conditions of 37°C and 5% CO₂. After the culture, the cell microfiber was washed once using a physiological saline. After the washing, the medium for the cell microfiber was replaced with the same alveolus differentiation medium as that in Example 1(8) above. The cell microfiber was cultured in the alveolus differentiation medium for 2 weeks. The media were replaced with new media every other day (the same applies hereinafter). In the differentiation induction, fibroblast cells serving as a feeder were not added.

In order to evaluate differentiation of the hLPs in the cell microfiber into alveolar epithelial cells, the rate of SFTPC-GFP positive cells was examined using flow cytometry. The preparation of the single cell suspension and the flow cytometry were performed in the same manner as in Example 1(8) above. Then, the rate of the SFTPC-GFP positive cells in the cell population with gating of EPCAM positive cells being performed. As a result, the rate of the SFTPC-GFP positive cells was 69.52%. A control was cultured in the same conditions, except that the cell microfiber was not used. In the case of the control, the rate of the SFTPC-GFP positive cells was 23.66%.

### (2) Condition 2

The hLPs isolated in Example 1(3) above were diluted to 5.0×10⁷ cells/ml using the same proliferation medium as that in Example 1(5) above or the same alveolus differentiation medium as that in Example 1(8) above. A cell microfiber that included the diluted cells was prepared in the same manner as in Example 1(5) above. The obtained cell microfiber was cultured in the proliferation medium for 3 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 1 week. In Reference Example 2(2), a cell microfiber was prepared using cells diluted in the alveolus differentiation medium, and was then cultured in the alveolus differentiation medium for 4 weeks. That is to say, in Reference Example 2(2), the proliferation of hLPs was omitted.

The rate of SFTPC-GFP positive cells was examined using flow cytometry in the same manner as in Example 2(1). As a result, the rate of the SFTPC-GFP positive cells was 3.56%. Meanwhile, in Reference Example 2(2), the rate of the SFTPC-GFP positive cells was 1.38%.

### (3) Condition 3

The hLPs isolated in Example 1(3) above were diluted to 2.0×10⁸ cells/ml using the same proliferation medium as that in Example 1(5) above or the same alveolus differentiation medium as that in Example 1(8) above. A cell microfiber that included the diluted cells was prepared in the same manner as in Example 1(5) above. The obtained cell microfiber was cultured in the proliferation medium for 2 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 2 weeks. In Reference Example 3(2), a cell microfiber was prepared using cells diluted in the alveolus differentiation medium, and was then cultured in the alveolus differentiation medium for 4 weeks. That is to say, in Reference Example 3(2), the proliferation of hLPs was omitted.

The rate of SFTPC-GFP positive cells was examined using flow cytometry in the same manner as in Example 2(1). As a result, the rate of the SFTPC-GFP positive cells was 6.05%. Meanwhile, in Reference Example 3(2), the rate of the SFTPC-GFP positive cells was 3.47%.

### (4) Condition 4

The hLPs isolated in Example 1(3) above were diluted to 5.0×10⁷ cells/ml using the same proliferation medium as that in Example 1(5) above. A cell microfiber that included the diluted cells was prepared in the same manner as in Example 1(5) above. The obtained cell microfiber was cultured in the proliferation medium for 2 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 1 week or 2 weeks.

The rate of SFTPC-GFP positive cells was examined using flow cytometry in the same manner as in Example 2(1). As a result, the rate of the SFTPC-GFP positive cells in the cells cultured in the alveolus differentiation medium for 1 week was 4.24%. Meanwhile, the rate of the SFTPC-GFP positive cells in the cells cultured in the alveolus differentiation medium for 2 weeks was 9.58%. Note that the expression of the type-II alveolar epithelial cell markers was confirmed in the cells cultured in the alveolus differentiation medium for 2 weeks through qRT-PCR. As shown in Example 2(1), the rate of the SFTPC-GFP positive cells in the cells cultured only in the alveolus differentiation medium for 4 weeks was 1.38%.

### (5) Condition 5

The hLPs isolated in Example 1(3) above were diluted to 1.0×10⁸ cells/ml using the same proliferation medium as that in Example 1(5) above. A cell microfiber that included the diluted cells was prepared in the same manner as in Example 1(5) above. The obtained cell microfiber was cultured in the proliferation medium for 10 days. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 6 days.

The rate of SFTPC-GFP positive cells was examined using flow cytometry in the same manner as in Example 2(1). As a result, the rate of the SFTPC-GFP positive cells in the cells cultured in the alveolus differentiation medium for 1 week was 3.52%.

### (6) Condition 6

The hLPs isolated in Example 1(3) above were diluted to 1.0×10⁷ cells/ml using the same proliferation medium as that in Example 1(5) above. A cell microfiber that included the diluted cells was prepared in the same manner as in Example 1(5) above. The obtained cell microfiber was cultured in the proliferation medium for 3 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 2 weeks.

The rate of SFTPC-GFP positive cells was examined using flow cytometry in the same manner as in Example 2(1). As a result, the rate of the SFTPC-GFP positive cells was 12.21%. Note that the expression of the type-II alveolar epithelial cell markers was confirmed in the induced type-II alveolar epithelial cells through qRT-PCR.

### (7) Condition 7

The hLPs isolated in Example 1(3) above were diluted to 1.0×10⁸ cells/ml using the same proliferation medium as that in Example 1(5) above. A cell microfiber that included the diluted cells was prepared in the same manner as in Example 1(5) above. The obtained cell microfiber was cultured in the proliferation medium for 1 week. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 1 week or 2 weeks.

The rate of SFTPC-GFP positive cells was examined using flow cytometry in the same manner as in Example 2(1). As a result, in the case of the culture in the alveolus differentiation medium for 1 week, the rate of the SFTPC-GFP positive cells was 5.12%. Meanwhile, in the case of the culture in the alveolus differentiation medium for 1 week, the rate of the SFTPC-GFP positive cells was 2.55%.

### (8) Condition 8

The hLPs isolated in Example 1(3) above were diluted to 2.0×10⁶ cells/ml using the same proliferation medium as that in Example 1(5) above. A cell microfiber that included the diluted cells was prepared in the same manner as in Example 1(5) above. The obtained cell microfiber was cultured in the proliferation medium for 8 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 2 weeks.

The rate of SFTPC-GFP positive cells was examined using flow cytometry in the same manner as in Example 2(1). As a result, the rate of the SFTPC-GFP positive cells was 53.27%.

### (9) Condition 9

The hLPs isolated in Example 1(3) above were diluted to 1.0×10⁷ cells/ml using the same proliferation medium as that in Example 1(5) above. A cell microfiber that included the diluted cells was prepared in the same manner as in Example 1(5) above. The obtained cell microfiber was cultured in the proliferation medium for 4 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 1 week or 2 weeks.

The rate of SFTPC-GFP positive cells was examined using flow cytometry in the same manner as in Example 2(1). As a result, in the case of the culture in the alveolus differentiation medium for 1 week, the rate of the SFTPC-GFP positive cells was 34.66%. Meanwhile, in the case of the culture in the alveolus differentiation medium for 2 weeks, the rate of the SFTPC-GFP positive cells was 49.39%. Note that the expression of the type-II alveolar epithelial cell markers was confirmed in the cells cultured in the alveolus differentiation medium for 2 weeks through qRT-PCR.

### (10) Condition 10

The hLPs isolated in Example 1(3) above were diluted to 5.0×10⁶ cells/ml or 1.0×10⁷ cells/ml using the same proliferation medium as that in Example 1(5) above. A cell microfiber that included the diluted cells was prepared in the same manner as in Example 1(5) above. The obtained cell microfiber was cultured in the proliferation medium for 4 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 2 weeks.

The rate of SFTPC-GFP positive cells was examined using flow cytometry in the same manner as in Example 2(1). As a result, in the case where the cells diluted to 5.0×10⁶ cells/ml were used, the rate of the SFTPC-GFP positive cells was 14.26%. Meanwhile, in the case where the cells diluted to 1.0×10⁷ cells/ml were used, the rate of the SFTPC-GFP positive cells was 13.79%. Note that, in both conditions, the expression of the type-II alveolar epithelial cell markers was confirmed through qRT-PCR.

### (11) Condition 11

The hLPs isolated in Example 1(3) above were diluted to 1.0×10⁷ cells/ml or 5.0×10⁷ cells/ml using the same proliferation medium as that in Example 1(5) above. A cell microfiber that included the diluted cells was prepared in the same manner as in Example 1(5) above. The obtained cell microfiber was cultured in the proliferation medium for 4 weeks. After the culture, in the same manner as in Example 2(1), the cell microfiber was washed using a physiological saline, and then the medium was replaced with the alveolus differentiation medium. After the replacement, the cell microfiber was cultured in the alveolus differentiation medium for 2 weeks.

The rate of SFTPC-GFP positive cells was examined using flow cytometry in the same manner as in Example 2(1). As a result, in the case where the cells diluted to 1.0×10⁷ cells/ml were used, the rate of the SFTPC-GFP positive cells was 9.64%. Meanwhile, in the case where the cells diluted to 5.0×10⁷ cells/ml were used, the rate of the SFTPC-GFP positive cells was 6.56%.

It was confirmed from these results that lung progenitor cells proliferated in the cell microfiber at various cell densities and during various culturing periods. When the hLP cell density was reduced at the time of the preparation of the cell microfiber, the rate of the type-II alveolar epithelial cells increased. Accordingly, it was suggested that, when the cell density of hLPs included in the cell microfiber is reduced at the time of the preparation of the cell microfiber, the cell proliferation of the hLPs is relatively promoted.

### Example 3

It was checked whether, in the case where the cell microfiber included lung progenitor cells and a matrigel, the lung progenitor cells proliferated and differentiated into an alveolar epithelial cell and an airway epithelial cell.

The hLPs isolated in Example 1(3) above were diluted to 5.0×10³ cells/ml using a mixed solution obtained by mixing the proliferation medium in Example 1(5) above and a matrigel in equal amounts. A cell microfiber that included the diluted cells was prepared in the same manner as in Example 1(5) above. The obtained cell microfiber was cultured in the proliferation medium for 2 weeks. After the culture, in the same manner as in Example 2(1), the cell microfiber was washed using a physiological saline, and then the medium was replaced with the alveolus differentiation medium or the airway differentiation medium. After the replacement with the alveolus differentiation medium, the cell microfiber was cultured in the alveolus differentiation medium for 2 weeks. After the culture, SFTPC and the nuclei were stained in the same manner as in Example 1(11) above. After the staining, the sample was observed using the fluorescent microscope. Also, the cells that had been cultured in the alveolus differentiation medium were observed using the transmission electron microscope in the same manner as in Example 1(12) above. As a result, it was confirmed that, in the cells that had been cultured in the alveolus differentiation medium, the type-II alveolar epithelial cell marker SFTPC was expressed, and lamellar bodies were formed. Also, it was confirmed that the lung progenitor cells proliferated because the inside of the cell microfiber was filled with the cells.

After the replacement with the airway differentiation medium, the cell microfiber was cultured in the airway differentiation medium for 4 weeks. After the culture, FOXJ1, acetylated-tubulin, p63, MUC5AC, and the nuclei were stained in the same manner as in Example 1(15) above. After the staining, the sample was observed using the fluorescent microscope. As a result, it was confirmed that the cells that had been cultured in the airway differentiation medium included airway epithelial cells expressing FOXJ1 and acetylated-tubulin, basal cells expressing p63, and mucilage cells expressing MUC. Also, it was confirmed that the lung progenitor cells proliferated because the inside of the cell microfiber was filled with the cells.

It was found from the results above that, in the case as well where the cell microfiber included lung progenitor cells and a matrigel, the lung progenitor cells also proliferated and differentiated into an alveolar epithelial cell and an airway epithelial cell.

As described above, the present invention has been described with reference to the embodiments and the examples, but the present invention is not limited to the above-described embodiments and examples. Various modifications that can be understood by a person skilled in the art can be made in the configurations and details of the present invention without departing from the scope of the present invention.

The present application claims the benefit of priority from Japanese Patent Application No. 2021-117466 filed on July 15, 2021, the entire disclosure of which is incorporated herein.

### Supplementary Notes

Some or all of the embodiments and the examples given above can be described as in the following supplementary notes. However, the scope of the present invention is not limited thereto.

### <First Structure>

### (Supplementary Note 1)

A structure including:
a core portion that includes a cell population; and
an outer shell portion that covers at least a part of the core portion and contains a hydrogel,
wherein the structure has a storage elastic modulus of 150 Pa or more and/or the hydrogel contains alginic acid, and
the cell population contains an alveolar epithelial cell, an airway epithelial cell, and/or a progenitor cell thereof.

### (Supplementary Note 2)

The structure according to supplementary note 1, wherein the cell population is included at a density of 1×10³ cells/core portion-mL or more.

### (Supplementary Note 3)

The structure according to supplementary note 1 or 2, wherein the cell population is included at a density of 1×10⁶ cells/core portion-mL or more.

### (Supplementary Note 4)

The structure according to any one of supplementary notes 1 to 3, wherein the cell population is included at a density of 5×10⁹ cells/core portion-mL or less.

### (Supplementary Note 5)

The structure according to any one of supplementary notes 1 to 4, wherein the progenitor cell includes a lung progenitor cell, a ventral anterior foregut endoderm cell, an anterior foregut endoderm cell, and/or a definitive endoderm cell.

### (Supplementary Note 6)

The structure according to any one of supplementary notes 1 to 5, wherein the progenitor cell is positive for carboxypeptidase M (CPM), NK2 homeobox 1 (NKX2.1), SRY-box 9 (SOX) 9, SRY-box 2 (SOX2), and/or forkhead box protein 2A (FOXA2).

### (Supplementary Note 7)

The structure according to supplementary note 6, wherein the progenitor cell is a lung progenitor cell that is positive for CPM and/or NKX2.1.

### (Supplementary Note 8)

The structure according to any one of supplementary notes 1 to 7, wherein the progenitor cell is induced from a pluripotent stem cell.

### (Supplementary Note 9)

The structure according to supplementary note 8, wherein the pluripotent stem cell is an induced pluripotent stem cell.

### (Supplementary Note 10)

The structure according to any one of supplementary notes 1 to 9, wherein the cell population is included as a cell suspension containing a cell or a spheroid.

### (Supplementary Note 11)

The structure according to any one of supplementary notes 1 to 10, wherein the hydrogel contains alginic acid, agarose, a chitosan gel, a collagen gel, gelatin, a peptide gel, or a fibrin gel, or a mixture thereof.

### (Supplementary Note 12)

The structure according to any one of supplementary notes 1 to 11, wherein the core portion does not contain the hydrogel.

### (Supplementary Note 13)

The structure according to any one of supplementary notes 1 to 12, wherein the structure is a columnar structure or a hollow structure.

### (Supplementary Note 14)

The structure according to any one of supplementary notes 1 to 13, wherein the structure has a fibrous shape, a spherical shape, or a capsule shape.

### <Cell Population>

### (Supplementary Note 15)

A cell population isolated from the structure according to any one of supplementary notes 1 to 14.

### (Supplementary Note 16)

The cell population according to supplementary note 15, having an ability to differentiate into an alveolar epithelial cell and/or an airway epithelial cell.

### (Supplementary Note 17)

The cell population according to supplementary note 15 or 16, having an improved ability to differentiate into an alveolar epithelial cell compared with a CPM positive lung progenitor cell.

### <Second Structure>

### (Supplementary Note 18)

A structure including:
a core portion that includes a cell population; and
an outer shell portion that covers at least a part of the core portion and contains a hydrogel,
wherein the structure has a storage elastic modulus of 150 Pa or more and/or the hydrogel contains alginic acid, and
the cell population forms a construct with functions of an alveolar epithelium and/or an airway epithelium, or is capable of forming the construct after an alveolar epithelium inducing test below or an airway epithelium inducing test below:
   alveolar epithelium inducing test: culture is performed at 37°C for 4 to 21 days in the presence of an alveolar epithelium inducing medium containing 50-nmmol/l dexamethasone, 100-µmmol/l 8-Br-cAMP, 100-µmmol/l 3-isobutyl-1-methylxanthine, 3-µmmol/l CHIR99021, 10-µmmol/l SB43154, and 10-µmmol/l Y-27632;
   airway epithelium inducing test: culture is performed at 37°C for 14 to 42 days in the presence of an airway epithelium inducing medium containing 4µg/ml heparin, 1-µmmol/l hydroxycortisone, 10-µmmol/l N-[N-(3,5-difluorophenacetyl-L-alanyl)]-(S)-phenylglycine t-butyl ester (DAPT), and 10-µmmol/l Y-27632.

### (Supplementary Note 19)

The structure according to supplementary note 18, wherein the cell population is included at a density of 1×10³ cells/core portion-mL or more.

### (Supplementary Note 20)

The structure according to supplementary note 18 or 19, wherein the cell population is included at a density of 1×10⁶ cells/core portion-mL or more.

### (Supplementary Note 21)

The structure according to any one of supplementary notes 18 to 20, wherein the cell population is included at a density of 5×10⁹ cells/core portion-mL or less.

### (Supplementary Note 22)

The structure according to any one of supplementary notes 18 to 21, wherein the progenitor cell includes a lung progenitor cell, a ventral anterior foregut endoderm cell, an anterior foregut endoderm cell, and/or a definitive endoderm cell.

### (Supplementary Note 23)

The structure according to any one of supplementary notes 18 to 22, wherein the progenitor cell is positive for carboxypeptidase M (CPM), NK2 homeobox 1 (NKX2.1), SRY-box 9 (SOX) 9, SRY-box 2 (SOX2), and/or forkhead box protein 2A (FOXA2).

### (Supplementary Note 24)

The structure according to supplementary note 23, wherein the progenitor cell is a lung progenitor cell that is positive for CPM and/or NKX2.1.

### (Supplementary Note 25)

The structure according to any one of supplementary notes 18 to 24, wherein the progenitor cell is induced from a pluripotent stem cell.

### (Supplementary Note 26)

The structure according to supplementary note 25, wherein the pluripotent stem cell is an induced pluripotent stem cell.

### (Supplementary Note 27)

The structure according to any one of supplementary notes 18 to 26, wherein the cell population is included as a cell suspension containing a cell or a spheroid.

### (Supplementary Note 28)

The structure according to any one of supplementary notes 18 to 27, wherein the hydrogel contains alginic acid, agarose, a chitosan gel, a collagen gel, gelatin, a peptide gel, or a fibrin gel, or a mixture thereof.

### (Supplementary Note 29)

The structure according to any one of supplementary notes 18 to 29, wherein the core portion does not contain the hydrogel.

### (Supplementary Note 30)

The structure according to any one of supplementary notes 18 to 29, wherein the structure is a columnar structure or a hollow structure.

### (Supplementary Note 31)

The structure according to any one of supplementary notes 18 to 30, wherein the structure has a fibrous shape, a spherical shape, or a capsule shape.

### <Construct>

### (Supplementary Note 32)

A construct isolated from the structure according to any one of supplementary notes 18 to 31.

### (Supplementary Note 33)

The construct according to supplementary note 32, which includes a lamellar body, and/or has an ability to secretes a pulmonary surfactant.

### (Supplementary Note 34)

The construct according to supplementary note 33, wherein the pulmonary surfactant is pulmonary surfactant protein B and/or pulmonary surfactant protein C.

### (Supplementary Note 35)

The construct according to any one of supplementary notes 32 to 34, including a cilium,
wherein the cilium has ciliary motility.

### <Method for Culturing Lung Progenitor Cell>

### (Supplementary Note 36)

A culturing method for proliferation or maintenance of a lung progenitor cell, comprising
a proliferation step of allowing the lung progenitor cell to proliferate by culturing a structure containing the lung progenitor cell in the presence of a proliferative factor for a lung progenitor cell,
wherein the structure includes:
   a core portion that includes a cell population; and
   an outer shell portion that covers at least a part of the core portion and contains a hydrogel,
   the structure has a storage elastic modulus of 150 Pa or more and/or the hydrogel contains alginic acid, and
   the cell population contains the lung progenitor cell.

### (Supplementary Note 37)

The culturing method according to supplementary note 36, wherein the cell population is included at a density of 1×10³ cells/core portion-mL or more.

### (Supplementary Note 38)

The culturing method according to supplementary note 36 or 37, wherein the cell population is included at a density of 1×10⁶ cells/core portion-mL or more.

### (Supplementary Note 39)

The culturing method according to any one of supplementary notes 36 to 38, wherein the cell population is included at a density of 5×10⁹ cells/core portion-mL or less.

### (Supplementary Note 40)

The culturing method according to any one of supplementary notes 36 to 39, wherein the lung progenitor cell is positive for carboxypeptidase M (CPM), NK2 homeobox 1 (NKX2.1), SRY-box 9 (SOX) 9, SRY-box 2 (SOX2), and/or forkhead box protein 2A (FOXA2).

### (Supplementary Note 41)

The culturing method according to supplementary note 40, wherein the progenitor cell is a lung progenitor cell that is positive for CPM and/or NKX2.1.

### (Supplementary Note 42)

The culturing method according to any one of supplementary notes 36 to 41, wherein the progenitor cell is induced from a pluripotent stem cell.

### (Supplementary Note 43)

The culturing method according to supplementary note 42, wherein the pluripotent stem cell is an induced pluripotent stem cell.

### (Supplementary Note 44)

The culturing method according to any one of supplementary notes 36 to 43, wherein the cell population is included as a cell suspension containing a cell or a spheroid.

### (Supplementary Note 45)

The culturing method according to any one of supplementary notes 36 to 44, wherein the hydrogel contains alginic acid, agarose, a chitosan gel, a collagen gel, gelatin, a peptide gel, or a fibrin gel, or a mixture thereof.

### (Supplementary Note 46)

The culturing method according to any one of supplementary notes 36 to 45, wherein the core portion does not contain the hydrogel.

### (Supplementary Note 47)

The culturing method according to any one of supplementary notes 36 to 46, wherein the culturing structure is a columnar structure or a hollow structure.

### (Supplementary Note 48)

The culturing method according to any one of supplementary notes 36 to 47, wherein the proliferative factor for a lung progenitor cell includes a GSK3β inhibitor, FGF10, KGF, a Notch inhibitor, and/or a ROCK inhibitor.

### (Supplementary Note 49)

The culturing method according to any one of supplementary notes 36 to 48, wherein, in the culturing step, the lung progenitor cell is cultured to fill the core portion.

### (Supplementary Note 50)

The culturing method according to any one of supplementary notes 36 to 49, which is an in-vitro or ex-vivo culturing method.

### <Method for Producing Cell>

### (Supplementary Note 51)

A method for producing a cell having an ability to differentiate into an alveolar epithelial cell or an airway epithelial cell, the method comprising
a proliferation step of allowing a cell having an ability to differentiate into an alveolar epithelial cell or an airway epithelial cell to proliferate by culturing a structure containing a lung progenitor cell,
wherein the proliferation step is the culturing method according to supplementary notes 36 to 50.

### <Method for Producing Alveolar Epithelial Cell>

### (Supplementary Note 52)

A method for producing an alveolar epithelial cell, comprising
an induction step of inducing an alveolar epithelial cell by culturing a structure containing a cell population in the presence of an inducing factor for an alveolar epithelial cell,
wherein the structure includes:
   a core portion that includes the cell population; and
   an outer shell portion that covers at least a part of the core portion and contains a hydrogel,
the structure has a storage elastic modulus of 150 Pa or more and/or the hydrogel contains alginic acid, and
the cell population contains a lung progenitor cell, and/or is capable of forming a construct with functions of an alveolar epithelium through culture in the induction step.

### (Supplementary Note 53)

The production method according to supplementary note 52, comprising a proliferation step of allowing the lung progenitor cell to proliferate by culturing the structure in the presence of a proliferative factor for a lung progenitor cell, prior to the induction step.

### (Supplementary Note 54)

The production method according to supplementary note 53, wherein the proliferative factor for a lung progenitor cell includes a GSK3β inhibitor, FGF 10, KGF, a Notch inhibitor, and/or a ROCK inhibitor.

### (Supplementary Note 55)

The production method according to any one of supplementary notes 52 to 54,
wherein the inducing factor for an alveolar epithelial cell is an inducing factor for a type-II alveolar epithelial cell, and
the inducing factor for a type-II alveolar epithelial cell is a steroid, a cAMP derivative, a phosphodiesterase inhibitor, KGF, a GSK3β inhibitor, a TGFβ inhibitor, a ROCK inhibitor, FGF10, and/or EGF.

### (Supplementary Note 56)

The production method according to any one of supplementary notes 52 to 54,
wherein the inducing factor for an alveolar epithelial cell is an inducing factor for a type-I alveolar epithelial cell, and
the inducing factor for a type-I alveolar epithelial cell is a Wnt inhibitor.

### <Method for Producing Airway Epithelial Cell>

### (Supplementary Note 57)

A method for producing an airway epithelial cell, comprising
an induction step of inducing an airway epithelial cell by culturing a structure containing a cell population in the presence of an inducing factor for an airway epithelial cell,
wherein the structure includes:
   a core portion that includes the cell population; and
   an outer shell portion that covers at least a part of the core portion and contains a hydrogel,
the structure has a storage elastic modulus of 150 Pa or more and/or the hydrogel contains alginic acid, and
the cell population contains a lung progenitor cell, and/or is capable of forming a construct with functions of an airway epithelium through culture in the induction step.

### (Supplementary Note 58)

The production method according to supplementary note 57, comprising a proliferation step of allowing the lung progenitor cell to proliferate by culturing the structure in the presence of a proliferative factor for a lung progenitor cell, prior to the induction step.

### (Supplementary Note 59)

The production method according to supplementary note 58, wherein the proliferative factor for a lung progenitor cell includes a GSK3β inhibitor, FGF10, KGF, a Notch inhibitor, and/or a ROCK inhibitor.

### (Supplementary Note 60)

The production method according to any one of supplementary notes 57 to 59, wherein the inducing factor for an airway epithelial cell is a steroid, a Notch inhibitor, a ROCK inhibitor, and/or heparin.

### Industrial Applicability

As described above, with the present invention, the lung progenitor cells can proliferate while retaining an ability to differentiate into an alveolar epithelial cell and an airway epithelial cell. Accordingly, the present invention is very useful in the fields of regenerative medicine, cell pharmaceutical drug, and the like.

### [Sequence Listing]

P22117WO Sequence Listing.xml

## Claims

1. A structure comprising:
a core portion that includes a cell population; and
an outer shell portion that covers at least a part of the core portion and contains a hydrogel,
wherein the structure has a storage elastic modulus of 150 Pa or more and/or the hydrogel contains alginic acid, and
the cell population contains an alveolar epithelial cell, an airway epithelial cell, and/or a progenitor cell thereof.

2. A structure comprising:
a core portion that includes a cell population; and
an outer shell portion that covers at least a part of the core portion and contains a hydrogel,
wherein the structure has a storage elastic modulus of 150 Pa or more and/or the hydrogel contains alginic acid, and
the cell population forms a construct with functions of an alveolar epithelium and/or an airway epithelium, or is capable of forming the construct after an alveolar epithelium inducing test below or an airway epithelium inducing test below:
alveolar epithelium inducing test: culture is performed at 37°C for 4 to 21 days in the presence of an alveolar epithelium inducing medium containing 50-nmmol/l dexamethasone, 100-µmmol/l 8-Br-cAMP, 100-µmmol/l 3-isobutyl-1-methylxanthine, 3-µmmol/l CHIR99021, 10-µmmol/l SB43154, and 10-µmmol/l Y-27632;
airway epithelium inducing test: culture is performed at 37°C for 14 to 42 days in the presence of an airway epithelium inducing medium containing 4-µg/ml heparin, 1-µmmol/l hydroxycortisone, 10-µmmol/l N-[N-(3,5-difluorophenacetyl-L-alanyl)]-(S)-phenylglycine t-butyl ester (DAPT), and 10-µmmol/l Y-27632.

3. The structure according to claim 1 or 2,
wherein the cell population is included at a density of 1×10³ cells/core portion-mL or more.

4. The structure according to any one of claims 1 to 3,
wherein the cell population is included at a density of 1×10⁶ cells/core portion-mL or more.

5. The structure according to any one of claims 1 to 4,
wherein the cell population is included at a density of 5×10⁹ cells/core portion-mL or less.

6. The structure according to any one of claims 1 to 5,
wherein the progenitor cell includes a lung progenitor cell, a ventral anterior foregut endoderm cell, an anterior foregut endoderm cell, and/or a definitive endoderm cell.

7. The structure according to any one of claims 1 to 5,
wherein the progenitor cell is positive for carboxypeptidase M (CPM), NK2 homeobox 1 (NKX2.1), SRY-box 9 (SOX) 9, SRY-box 2 (SOX2), and/or forkhead box protein 2A (FOXA2).

8. The structure according to claim 7,
wherein the progenitor cell is a lung progenitor cell that is positive for CPM and/or NKX2.1.

9. The structure according to any one of claims 1 to 8,
wherein the cell population is included as a cell suspension containing a cell or a spheroid.

10. The structure according to any one of claims 1 to 9,
wherein the hydrogel contains alginic acid, agarose, a chitosan gel, a collagen gel, gelatin, a peptide gel, or a fibrin gel, or a mixture thereof.

11. The structure according to any one of claims 1 to 10,
wherein the core portion does not contain the hydrogel.

12. The structure according to any one of claims 1 to 11,
wherein the structure is a columnar structure or a hollow structure.

13. A cell population or a construct isolated from the structure according to any one of claims 1 to 12.

14. A culturing method for proliferation or maintenance of a lung progenitor cell, comprising
a proliferation step of allowing the lung progenitor cell to proliferate by culturing a structure containing the lung progenitor cell in the presence of a proliferative factor for a lung progenitor cell,
wherein the structure includes:
a core portion that includes a cell population; and
an outer shell portion that covers at least a part of the core portion and contains a hydrogel,
the structure has a storage elastic modulus of 150 Pa or more and/or the hydrogel contains alginic acid, and
the cell population contains the lung progenitor cell.

15. The culturing method according to claim 14,
wherein the cell population is included at a density of 1×10³ cells/core portion-mL or more.

16. The culturing method according to claim 14 or 15,
wherein the cell population is included at a density of 1×10⁶ cells/core portion-mL or more.

17. The culturing method according to any one of claims 14 to 16,
wherein the cell population is included at a density of 5×10⁹ cells/core portion-mL or less.

18. The culturing method according to any one of claims 14 to 17,
wherein the lung progenitor cell is positive for carboxypeptidase M (CPM), NK2 homeobox 1 (NKX2.1), SRY-box 9 (SOX) 9, SRY-box 2 (SOX2), and/or forkhead box protein 2A (FOXA2).

19. The culturing method according to claim 18,
wherein the progenitor cell is a lung progenitor cell that is positive for CPM and/or NKX2.1.

20. The culturing method according to any one of claims 14 to 19,
wherein the cell population is included as a cell suspension containing a cell or a spheroid.

21. The culturing method according to any one of claims 14 to 20,
wherein the hydrogel contains alginic acid, agarose, a chitosan gel, a collagen gel, gelatin, a peptide gel, or a fibrin gel, or a mixture thereof.

22. The culturing method according to any one of claims 14 to 21,
wherein the core portion does not contain the hydrogel.

23. The culturing method according to any one of claims 14 to 22,
wherein the culturing structure is a columnar structure or a hollow structure.

24. The culturing method according to any one of claims 14 to 23,
wherein the proliferative factor for a lung progenitor cell includes a GSK3β inhibitor, FGF10, KGF, a Notch inhibitor, and/or a ROCK inhibitor.

25. The culturing method according to any one of claims 14 to 24,
wherein, in the culturing step, the lung progenitor cell is cultured to fill the core portion.

26. A method for producing an alveolar epithelial cell, comprising
an induction step of inducing an alveolar epithelial cell by culturing a structure containing a cell population in the presence of an inducing factor for an alveolar epithelial cell,
wherein the structure includes:
a core portion that includes the cell population; and
an outer shell portion that covers at least a part of the core portion and contains a hydrogel,
the structure has a storage elastic modulus of 150 Pa or more and/or the hydrogel contains alginic acid, and
the cell population contains a lung progenitor cell, and/or is capable of forming a construct with functions of an alveolar epithelium through culture in the induction step.

27. The production method according to claim 26, comprising
a proliferation step of allowing the lung progenitor cell to proliferate by culturing the structure in the presence of a proliferative factor for a lung progenitor cell, prior to the induction step.

28. The production method according to claim 27,
wherein the proliferative factor for a lung progenitor cell includes a GSK3β inhibitor, FGF10, KGF, a Notch inhibitor, and/or a ROCK inhibitor.

29. The production method according to any one of claims 26 to 28,
wherein the inducing factor for an alveolar epithelial cell is an inducing factor for a type-II alveolar epithelial cell, and
the inducing factor for a type-II alveolar epithelial cell is a steroid, a cAMP derivative, a phosphodiesterase inhibitor, KGF, a GSK3β inhibitor, a TGFβ inhibitor, a ROCK inhibitor, FGF10, and/or EGF.

30. The production method according to any one of claims 26 to 28,
wherein the inducing factor for an alveolar epithelial cell is an inducing factor for a type-I alveolar epithelial cell, and
the inducing factor for a type-I alveolar epithelial cell is a Wnt inhibitor.

31. A method for producing an airway epithelial cell, comprising
an induction step of inducing an airway epithelial cell by culturing a structure containing a cell population in the presence of an inducing factor for an airway epithelial cell,
wherein the structure includes:
a core portion that includes the cell population; and
an outer shell portion that covers at least a part of the core portion and contains a hydrogel,
the structure has a storage elastic modulus of 150 Pa or more and/or the hydrogel contains alginic acid, and
the cell population contains a lung progenitor cell, and/or is capable of forming a construct with functions of an airway epithelium through culture in the induction step.

32. The production method according to claim 31, comprising
a proliferation step of allowing the lung progenitor cell to proliferate by culturing the structure in the presence of a proliferative factor for a lung progenitor cell, prior to the induction step.

33. The production method according to claim 32,
wherein the proliferative factor for a lung progenitor cell includes a GSK3β inhibitor, FGF10, KGF, a Notch inhibitor, and/or a ROCK inhibitor.

34. The production method according to any one of claims 31 to 33,
wherein the inducing factor for an airway epithelial cell is a steroid, a Notch inhibitor, a ROCK inhibitor, and/or heparin.
